(19) Europäisches Patentamt
European Patent Office
Office européen des brevets

(11) EP 4 115 975 A1

(12) **EUROPEAN PATENT APPLICATION**
published in accordance with Art. 153(4) EPC

(43) Date of publication:
**11.01.2023 Bulletin 2023/02**

(21) Application number: **21765247.8**

(22) Date of filing: **02.03.2021**

(51) International Patent Classification (IPC):
***B01J 31/22*** (2006.01) ***C07B 53/00*** (2006.01)
***C07C 29/159*** (2006.01) ***C07C 31/125*** (2006.01)
***C07C 33/22*** (2006.01) ***C07C 33/46*** (2006.01)
***C07C 41/26*** (2006.01) ***C07C 43/23*** (2006.01)
***C07C 253/30*** (2006.01) ***C07C 255/53*** (2006.01)
***C07B 61/00*** (2006.01)

(52) Cooperative Patent Classification (CPC):
**B01J 31/22; C07B 53/00; C07C 29/159; C07C 31/125; C07C 33/22; C07C 33/46; C07C 41/26; C07C 43/23; C07C 253/30; C07C 255/53;** C07B 61/00

(86) International application number:
**PCT/JP2021/007899**

(87) International publication number:
**WO 2021/177287 (10.09.2021 Gazette 2021/36)**

(84) Designated Contracting States:
**AL AT BE BG CH CY CZ DE DK EE ES FI FR GB GR HR HU IE IS IT LI LT LU LV MC MK MT NL NO PL PT RO RS SE SI SK SM TR**
Designated Extension States:
**BA ME**
Designated Validation States:
**KH MA MD TN**

(30) Priority: **03.03.2020 JP 2020036096**

(71) Applicants:
- **Takasago International Corporation**
  **Tokyo 144-8721 (JP)**
- **Osaka Gas Chemicals Co., Ltd.**
  **Osaka-shi, Osaka 550-0023 (JP)**

(72) Inventors:
- **YOKOYAMA Naota**
  **Hiratsuka-shi, Kanagawa 254-0073 (JP)**
- **TSUKADA Shinji**
  **Hiratsuka-shi, Kanagawa 254-0073 (JP)**
- **CHISHIRO Takefumi**
  **Osaka-shi, Osaka 550-0023 (JP)**
- **MIYAJI Yusuke**
  **Osaka-shi, Osaka 550-0023 (JP)**

(74) Representative: **Zimmermann & Partner Patentanwälte mbB**
**Postfach 330 920**
**80069 München (DE)**

(54) **CATALYST CONTAINING ACTIVATED CARBON ON WHICH RUTHENIUM COMPLEX IS ADSORBED, AND METHOD FOR PRODUCING REDUCTION PRODUCT USING SAME**

(57) The present invention addresses the problem of providing a catalyst which exhibits high reusability and is capable of reducing the amount of residual metals in a reaction liquid in a process wherein an optically active reduction product is produced by an asymmetric reduction reaction of an organic compound. The present invention relates to a catalyst that contains an activated carbon on which a ruthenium complex represented by general formula (1-1) and/or (1-2) is adsorbed. (In the formulae, j, k, X, Y, and $R^1$ to $R^{10}$ are as defined in claim 1.)

EP 4 115 975 A1

# FIG.4

CATALYST 17

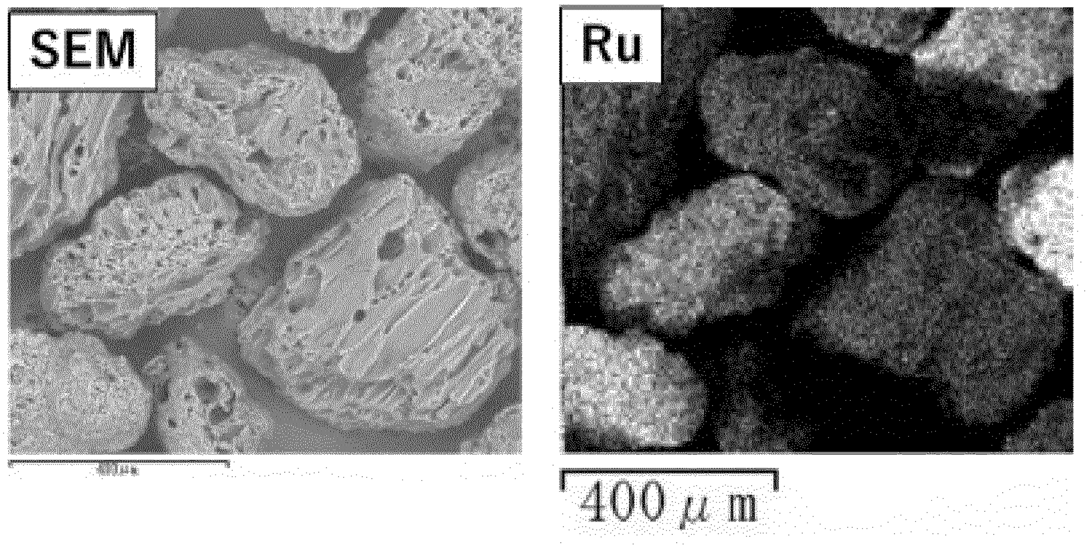


CATALYST 20

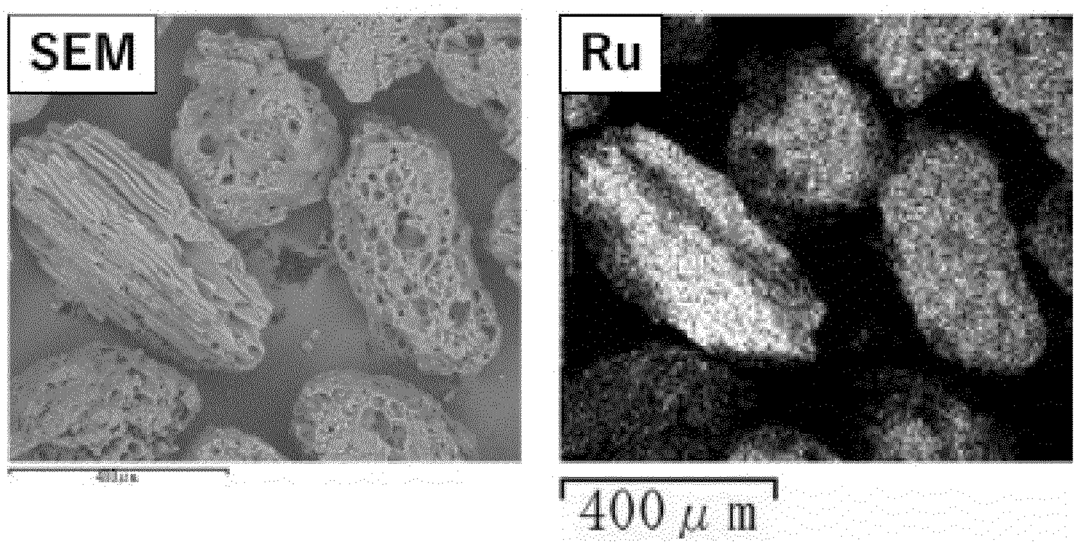

## Description

### Technical Field

The present invention relates to a novel catalyst containing activated carbon absorbed with a ruthenium complex, and a method for producing a reduction product including a step of reducing an organic compound using the same.

### Background Art

Many reports have been made that ruthenium complexes with chiral diamine ligands have excellent performance as homogeneous catalysts for an asymmetric hydrogenation. For example, the use of these catalysts makes it possible to efficiently produce an optically active alcohol by asymmetric hydrogenation of a ketone and an imine using molecular hydrogen, formic acid, formate, or the like as a hydrogen source. Among them, various chiral diamine ligands have been developed in order to enhance the performance of catalysts (see Patent Literatures 1, 2, and 3 and Non Patent Literatures 1 and 2).

Many efforts have been made to immobilize such ruthenium complexes on solid supports to work as heterogeneous catalysts. A complex supported on a solid phase support can impart features not found in the homogeneous catalyst, such as allowing reuse of the catalyst and allowing reduction of the amount of dissolved metal remaining after the reaction, and is expected to solve important problems such as reduction of environmental loads and improvement of manufacturing costs.

As a method for immobilizing a ruthenium complex for asymmetric hydrogenation, a method for cross-linking a ligand and a functional group on the surface of a support has been studied, and it has been found that supporting on silica (see Patent Literature 4 and Non Patent Literature 3), various polymers (see Non Patent Literature 4), and the like is effective.

Further, a method of chemically modifying the inside of the pores of a specific support to immobilize the complex without cross-linking the ligand of the complex and the surface of the support has been reported (see Non Patent Literature 5).

The above-mentioned support method requires new chemical modification of the conventional ruthenium complex or catalyst support, which leads to an increase in the cost of producing the desired optically active alcohol, and thus limits its industrial utility. Furthermore, depending on the catalyst and reaction substrate used, the catalyst efficiency and asymmetric yield may decrease due to immobilization on the support.

Activated carbon is known as a support that does not require chemical modification for supporting complexes or catalysts, and it has been reported that $RuCl_2(PPh_3)_3$ complex and a $[RuCl(p\text{-cymene})]_2$ complex supported on activated carbon are useful for the oxidation reaction of alcohols and diols (see Non Patent Literatures 6 and 7).

On the other hand, there is no example of synthesizing an activated carbon-supported complex useful for an asymmetric hydrogenation reaction. For example, activated carbon-supported ruthenium nanoparticle catalysts, which are effective supported ruthenium catalysts for hydrogenation of ketones, give racemic alcohols (see Non Patent Literature 8).

### Citation List

#### Patent Literatures

Patent Literature 1: Japanese Patent No. 3040353
Patent Literature 2: Japanese Patent No. 5718178
Patent Literature 3: Japanese Patent No. 5727127
Patent Literature 4: International Publication No. WO2016-056669


#### Non Patent Literatures

Non Patent Literature 1: Acc. Chem. Res. 30 (1997) p. 97-102
Non Patent Literature 2: J. Am. Chem. Soc. 133 (2011) p. 14960-14963
Non Patent Literature 3: Org. Lett. 6 (2004) p. 169-172
Non Patent Literature 4: Tetrahedron Asymmetry 9 (1998) p. 2015-2018

Non Patent Literature 5: Chem. Commun. 46 (2010) p. 8145-8147
Non Patent Literature 6: Org. Lett. 1 (1999) p. 713-715
Non Patent Literature 7: Org. Lett. 4 (2002) p. 2369-2371
Non Patent Literature 8: Org. Process Res. Dev. 22 (2018) p. 1580-1585

## Summary of Invention

### Problems to be solved by the invention

An object of the present invention is to provide a catalyst that exhibits high reusability and can reduce the amount of residual metal in a reaction solution in a method for producing an optically active reduction product by an asymmetric hydrogenation of an organic compound. Another object of the present invention is to provide the catalyst by a simple method that does not require chemical modification of the complex and the support.

### Means for solution of the problems

In order to achieve the above objects, the present inventors earnestly studied the support of a complex on a solid phase support, and have found as a result that the use of activated carbon makes it possible to support a ruthenium complex with a chiral diamine ligand by a simple method.
Furthermore, it has been found that a catalyst containing activated carbon adsorbed with a ruthenium complex according to the present invention provides a comparable catalytic efficiency and an equivalent asymmetric yield, compared to those of a complex catalyst for an asymmetric hydrogenation of ketones. In the catalyst of the present invention, almost no metal elution into the reaction solution is observed. Furthermore, it has been found that there is almost no decrease in catalytic efficiency or asymmetric yield in the asymmetric hydrogenation even in the repeated use. These findings have led to the completion of the present invention.
That is, the present invention includes the following contents.

[1] A catalyst comprising activated carbon adsorbed with a ruthenium complex, represented by the following general formula (1-1) and/or (1-2),

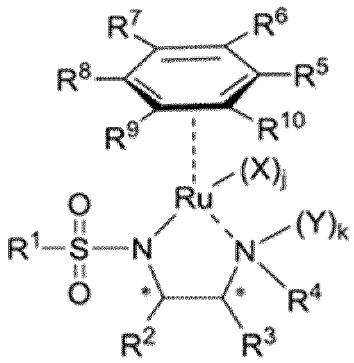

(1-1)

j = k = 1

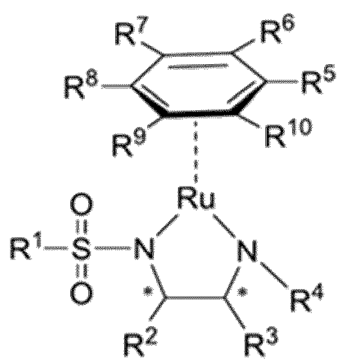

(1-2)

wherein

a solid line indicates a single bond, a double line indicates a double bond, and a broken line indicates a coordination bond;
Ru represents a ruthenium atom, N represents a nitrogen atom, S represents a sulfur atom, and O represents an oxygen atom;
* represents an asymmetric carbon atom;
j and k are integers of 0 or 1, where j+k does not become 1, and X represents an anionic group and Y represents a hydrogen atom;
$R^1$ represents a linear or branched alkyl group having 1 to 10 carbon atoms; a 10-camphalyl group; an aryl group which may be substituted with an alkyl group having 1 to 10 carbon atoms, an alkyl halide group having 1 to 10 carbon atoms, a halogen atom, a cyano group (-CN), an amino group, an alkylamino group ($-NR^{11}R^{12}$), a 5-membered or 6-membered cyclic amino group, an acylamino group ($-NH-CO-R^{11}$), a hydroxyl group, an

alkoxy group (-$OR^{11}$), an acyl group (-CO-$R^{11}$), a carboxyl group, an alkoxy carbonyl group (-$COOR^{11}$), a phenoxycarbonyl group, or an alkylthio group (-$SR^{11}$); or an aralkyl group which may be substituted with an alkyl group having 1 to 10 carbon atoms;
$R^{11}$ and $R^{12}$ each independently represent a hydrogen atom; an alkyl group having 1 to 10 carbon atoms; or a cycloalkyl group having 3 to 10 carbon atoms;
$R^2$ and $R^3$ each independently represent a hydrogen atom; or a phenyl group which may be substituted with an alkyl group having 1 to 10 carbon atoms, an alkoxy group having 1 to 10 carbon atoms, or a halogen atom, or $R^2$ and $R^3$ are bonded to each other to form a 4- to 8-membered cycloalkane ring with the carbon atom to which $R^2$ and $R^3$ are bonded;
$R^4$ represents a hydrogen atom; or a linear or branched alkyl group having 1 to 10 carbon atoms which may have a substituent;
$R^5$ to $R^{10}$ each independently represent a hydrogen atom; a linear or branched alkyl group having 1 to 10 carbon atoms; a hydroxyl group; or a linear or branched alkoxy group having 1 to 10 carbon atoms; and
$R^4$ and $R^5$ may be bonded to each other to form a cross-linking site of a divalent group represented by the following formula (W) to form a cross-linking site (W),

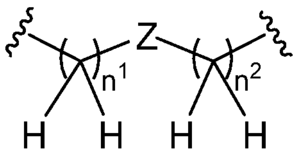

(W)

where

a wavy line section at a carbon chain terminal containing $n^1$ is bonded to a carbon atom of an arene moiety instead of $R^5$ in the formulas (1-1) and (1-2), and a wavy line section at a carbon chain terminal containing $n^2$ in the formula (W) is bonded to a nitrogen atom of an amine moiety instead of $R^4$ in the formulas (1-1) and (1-2);
Z represents a methylene group or an oxygen atom;
$n^1$ is an integer of 1 or 2; and
$n^2$ is an integer of 1, 2, or 3.

[2] The catalyst according to the above [1], wherein

$R^4$ and $R^5$ are bonded to each other to form the cross-linking site of the divalent group represented by the above formula (W),
Z is an oxygen atom,
$n^1$ is 1, and
$n^2$ is 2.

[3] The catalyst according to the above [2], wherein

$R^1$ is a 4-methylphenyl group or a methyl group,
$R^2$ and $R^3$ are phenyl groups,
$R^6$, $R^7$, $R^9$, and $R^{10}$ are hydrogen atoms, and
$R^8$ is a methyl group.

[4] The catalyst according to the above [1], wherein

$R^4$ and $R^5$ do not form the cross-linking site of the divalent group represented by the above formula (W), and $R^4$ is a hydrogen atom.

[5] The catalyst according to the above [4], wherein

$R^1$ is a 4-methylphenyl group, a 2,3,4,5,6-pentafluorophenyl group, a methyl group, an isobutyl group, a benzyl group, a 2',5'-dimethylbenzyl group, or a 10-camphalyl group,
$R^2$ and $R^3$ are phenyl groups or bonded to each other to form a cyclohexane ring, and
$R^5$, $R^6$, $R^7$, $R^8$, $R^9$ and $R^{10}$ are hydrogen atoms, methyl groups, or isopropyl groups.

[6] The catalyst according to any one of the above [1] to [5], wherein a total mass of the ruthenium complex is 0.1% by mass or more and 25% by mass or less based on a total mass of the activated carbon.

[7] The catalyst according to any one of the above [1] to [6], wherein the activated carbon has a specific surface area of 800 $m^2/g$ or more and 2000 $m^2/g$ or less.

[8] A method for producing a reduction product, comprising: a step of reducing an organic compound in the presence of the catalyst according to any one of claims 1 to 7 and a hydrogen donor.

[9] A method for producing an optically active alcohol, comprising: a step of reducing a carbonyl group of a carbonyl compound in the presence of the catalyst according to any one of the above [1] to [7] and a hydrogen donor.

[10] A method for producing an optically active amine, comprising: a step of reducing an imino group of an imine compound in the presence of the catalyst according to any one of the above [1] to [7] and a hydrogen donor.

[11] The production method according to any one of the above [8] to [10], wherein the hydrogen donor is at least one selected from the group consisting of formic acid, an alkali metal formate, an alcohol having a hydrogen atom at an $\alpha$-position carbon atom of a hydroxyl group substituted carbon, and hydrogen gas.

In the present invention, "adsorption" and "support" are used as having the same definition.

Advantageous Effects of Invention

The present invention makes it possible to provide a catalyst that exhibits high reusability and can reduce the amount of residual metal in a reaction solution in a method for producing an optically active reduction product by an asymmetric hydrogenation of an organic compound. In addition, the present invention makes it possible to provide the catalyst by a simple method that does not require chemical modification of the complex and the support.

Brief Description of Drawings

Fig. 1 is a graph showing changes over time in conversion rate examined in Example 32.

Fig. 2 is a graph showing changes over time in conversion rate examined in Examples 32 and 33.

Fig. 3 is a chart showing the analysis results of $^1H$ NMR in the coexistence of a (R,R)-Ts-DENEB complex and a $\pi$-conjugated compound.

Fig. 4 provides SEM images and ruthenium element mapping images of catalyst 17 and catalyst 19 taken by SEM-EDS.

Description of Embodiments

[Catalyst]

A catalyst of the present invention is characterized by containing activated carbon as a support on which a tether-type ruthenium complex and/or non-tether type ruthenium complex represented by the formulas (1-1) and/or (1-2) described later is adsorbed. The tether-type ruthenium complex and/or non-tether-type ruthenium complex used in the present invention is described later. These ruthenium complexes have a ligand having a benzene ring as a basic structure. In the catalyst according to the present invention, it is considered that the activated carbon and the ruthenium complex each have an aromatic ring, particularly a benzene ring structure, interacting with each other and adsorbing each other. This is also corroborated by the results shown in Fig. 3, that is, in the coexistence of a (R,R)-Ts-DENEB and a $\pi$-conjugated compound, an interaction occurs between the aromatic rings on ruthenium and the $\pi$-conjugated compound, and the peak (4.5 ppm to 6.2 ppm) attributed to the protons around the aromatic rings on ruthenium shifts to the high magnetic field side.

Further, in the catalyst of the present invention, the total mass of the ruthenium complex is not particularly limited, but preferably in the range of 0.1% by mass to 25% by mass, and preferably 1% by mass to 10% by mass, based on the total mass of the activated carbon, because it is possible to leverage the desired function as a catalyst and suppress the amount of ruthenium eluted from the catalyst of the present invention after the reaction, improving reusability. Further, in the catalyst of the present invention, it is more preferable that the ruthenium complex is uniformly supported on the surface of the activated carbon or the inside thereof.

Here, in the present invention, the loading amount (or adsorption amount) of the complex based on the total mass of the activated carbon in the catalyst can also be a value calculated from the amount of unadsorbed complex measured by analysis in the manufacturing process as in the present examples, but as an objective value, it is possible to employ a value calculated by surface analysis of activated carbon by SEM-EDS as in analysis 2 in the present examples

as the loading amount.

In the complex of the present invention, neither the ligand of the ruthenium complex nor the activated carbon as a support need to be chemically modified to bind to each other, so that there is an advantage that the synthesis is easier than the conventionally used ones, such as a ruthenium complex supported on silica or a ruthenium complex supported on a polymer. The present catalyst contains activated carbon as a support and can be recovered in a state where the ruthenium complex is adsorbed on the activated carbon, so that it is highly reusable. Here, in the catalyst of the present invention, the ruthenium complex is more preferably a tether type. This is because it tends to have high activity as a catalyst since there is a covalent bond between the benzene ring moiety and the diamine moiety on ruthenium, suppressing the release of the ligand from ruthenium.

The composition of the catalyst of the present invention containing a ruthenium complex adsorbed on activated carbon improves its catalytic activity, and tends to improve the obtained optical purity when used, for example, in the production of chiral alcohols. Further, when the catalyst according to the present invention is contained in a highly polar solvent such as water or ethanol, the interaction between the activated carbon and the ruthenium complex tends to be stronger.

Hereinafter, the activated carbon and the ruthenium complex constituting the catalyst according to the present invention will be described, and then a method for adsorbing the ruthenium complex to the activated carbon will be described.

[Activated Carbon]

The specific surface area of the activated carbon as a support in the present invention is generally 700 to 3000 $m^2/g$, preferably 800 to 2000 $m^2/g$, and more preferably 1200 to 1800 $m^2/g$. The specific surface area of the activated carbon used in the present invention affects the amount of the target complex adsorbed. In the present invention, the specific surface area of the activated carbon can be, for example, a value measured by the method specified in JIS Z8830: 2013 (ISO 9277: 2010).

Since the average pore diameter of the adsorbent of the present invention affects the reaction activity of the catalyst, it is preferably 1.0 to 5.0 nm, and more preferably 1.5 to 4.0 nm. The average pore diameter of the adsorbent of the present invention is obtained from the pore volume and the specific surface area obtained from the amount of nitrogen adsorbed.

Activated carbon usually has an acidic group on its surface. There are various acidic groups on the activated carbon surface which can be evaluated by measuring their amount. Since the acidic group affects the reactivity of the catalyst, it is preferable that the acid component is as small as possible, although there is no particular limitation, and specifically, it is preferable that the acid component in the activated carbon measured with sodium hydroxide is small.

From the above viewpoint, in the adsorbent of the present invention, the amount of acidic group measured with sodium hydroxide is preferably 0.50 mmol/g or less, more preferably 0.20 mmol/g or less, and further preferably 0.10 mmol/g or less.

As to the shape of the adsorbent of the present invention, any of granular, powdery, and fibrous can be employed. That is, when the adsorbent of the present invention is made of activated carbon, any of granular activated carbon, powdery activated carbon, and fibrous activated carbon can be employed. Note that the granular activated carbon means activated carbon having a particle diameter of 0.150 mm or more specified in JIS K1474. Here, a particle diameter of 0.150 mm or more, as specified by JIS K1474, has the same definition with a granularity of 0.150 mm or more measured by JIS K1474, and specifically, a sample having a granularity range of 0.150 mm or more has a mass fraction of 95% or more. On the other hand, the powdery activated carbon means that the particle diameter specified in JIS K1474 is less than 0.150 mm. Further, the fibrous activated carbon means activated carbon having a fibrous shape.

The average particle diameter of the adsorbent of the present invention is not particularly limited, and activated carbon of various granularities can be used, but it can be preferably 0.1 $\mu$m to 10000 $\mu$m, and more preferably 1 $\mu$m to 3000 $\mu$m, from the viewpoint of operability before and after the reaction and reaction efficiency.

The ignition residue contained in the activated carbon used in the support of the present invention is preferably 10% or less, and more preferably 1% or less, because there is a concern that an unexpected side reaction may occur in the catalytic reaction.

The method for activating the activated carbon of the present invention is not particularly limited. For example, it can be obtained by a production method including a step of performing a steam activation treatment or a chemical activation treatment using an activated carbon precursor.

The raw material for the activated carbon used in the support of the present invention is not particularly limited as long as it is a commonly used carbon source, and examples thereof include wood, wood flour, coconut shells, coal, and carbonized phenol resin. Among these, plant raw materials are preferable, and wood flour, coconut shells, and the like are more preferable, from the viewpoint of low impurity content.

The step after activation is not particularly limited, and any activated carbon produced through a known cleaning

step, heat treatment step, crushing and screening step necessary for satisfying the above characteristics can be used.

[Ruthenium Complex]

The ruthenium complexes represented by the general formulas (1-1) and (1-2) contained in the catalyst of the present invention will be described in detail. The ruthenium complexes represented by the general formulas (1-1) and (1-2) used in the present invention are characterized by having a diamine ligand, an aromatic compound (arene) ligand, and an anionic group.

In the general formulas (1-1) and (1-2) used in the present invention, a solid line indicates a single bond, a double line indicates a double bond, and a broken line indicates a coordination bond, and Ru represents a ruthenium atom, N represents a nitrogen atom, S represents a sulfur atom, and O represents an oxygen atom. The * mark in the general formulas (1-1) and (1-2) indicates that any carbon atom with the * mark may be an asymmetric carbon atom. If that carbon atom is an asymmetric carbon atom, it may be an optically active substance thereof, a mixture of the optically active substances, or a racemate (including a racemic compound). In a preferable embodiment of the present invention, if these carbon atoms are asymmetric carbon atoms, optically active substances thereof can be mentioned.

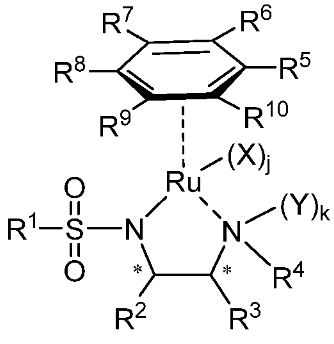

(1-1)

j = k = 1

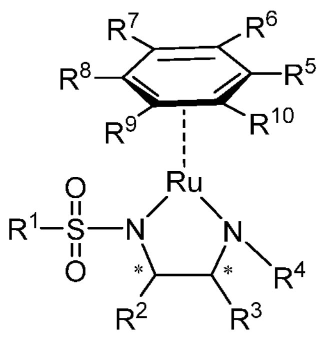

(1-2)

j = k = 0

In the general formula (1-1), j and k are integers of 0 or 1, where j+k does not become 1. The general formula (1-1) shows the case where j and k are 1, and the general formula (1-2) shows the case where j and k in the general formula (1-1) are 0. When j and k are 1, the bond between the ruthenium atom and the nitrogen atom is a coordinate bond shown by a broken line, and when j and k are 0, the bond between the ruthenium atom and the nitrogen atom is a covalent bond shown by a solid line.

In the general formula (1-1), X represents an anionic group and Y represents a hydrogen atom.

Examples of the anionic group represented by X in the general formula (1-1) include a trifluoromethane sulfonyloxy group, a p-toluenesulfonyloxy group, a methanesulfonyloxy group, a benzenesulfonyloxy group, a hydrogen atom, a halogen atom, and the like. Preferred X includes hydrogen atom and halogen atom, specifically, fluorine atom, chlorine atom, bromine atom, and the like, and for example, chlorine atom is particularly preferable.

The hydrogen atom in Y of the general formula (1-1) and the hydrogen atom at the position of X in the general formula (1-1) (that is, the hydrogen atoms bonded to Ru in the general formula (1-2)) may be not only a normal hydrogen atom but also an isotope of a hydrogen atom. Preferred isotopes include deuterium atoms.

<Sulfonyl Moiety ($R_1$)>

$R^1$ of the general formulas (1-1) and (1-2) represents an alkyl group; a 10-camphalyl group; an aryl group which may have a substituent; or an aralkyl group which may have a substituent.

<<Sulfonyl Moiety ($R^1$): Alkyl group>>

The alkyl group represented by $R^1$ of the general formulas (1-1) and (1-2) includes a linear or branched alkyl group having 1 to 10 carbon atoms, preferably 1 to 5 carbon atoms. Specific examples of the alkyl group include a methyl group, an ethyl group, an n-propyl group, an isopropyl group, an n-butyl group, an isobutyl group, an s-butyl group, a t-butyl group, an n-pentyl group, an n-hexyl group, an n-heptyl group, an n-octyl group, an n-nonyl group, an n-decyl group, and the like.

Further, the alkyl group represented by $R^1$ of the general formulas (1-1) and (1-2) may have one or more substituents selected from halogen atoms such as a fluorine atom, a chlorine atom, and a bromine atom. Examples

thereof include a perfluoroalkyl group such as a trifluoromethyl group, a pentafluoroethyl group, or a heptafluoropropyl group.

<<Sulfonyl Moiety ($R^1$): Aryl group which may have a substituent>>

The aryl group represented by $R^1$ of the general formulas (1-1) and (1-2) includes an aromatic monocyclic group, an aromatic polycyclic group, or an aromatic fused cyclic group, each having 6 to 30 carbon atoms, and preferably an aromatic monocyclic group, an aromatic polycyclic group, or an aromatic fused ring type group, each having 6 to 15 carbon atoms, and particularly preferably an aromatic monocyclic group having 6 to 12 carbon atoms. Specific examples of the aryl group having 6 to 30 carbon atoms include a phenyl group, a naphthyl group, an anthryl group, a phenanthryl group, an indenyl group, and the like, and a phenyl group is preferable.

Further, the aryl group represented by $R^1$ of the general formulas (1-1) and (1-2) may have one or more substituents selected from an alkyl group having 1 to 10 carbon atoms, an alkyl halide group having 1 to 10 carbon atoms, a halogen atom, a cyano group (-CN), an amino group, an alkylamino group ($-NR^{11}R^{12}$), 5-membered or 6-membered cyclic amino group, acylamino group ($-NH-CO-R^{11}$), hydroxyl group, alkoxy group ($-OR^{11}$), acyl group ($-CO-R^{11}$), carboxyl group, an alkoxy carbonyl group ($-COOR^{11}$), a phenoxycarbonyl group, and an alkylthio group ($-SR^{11}$).

The alkyl group as a substituent can be selected from the groups defined as the alkyl groups represented by $R^1$ in the general formulas (1-1) and (1-2) described above, and preferably includes a linear or branched alkyl group having 1 to 5 carbon atoms. Further, the alkyl group as a substituent may have one or more substituents selected from halogen atoms such as a fluorine atom, a chlorine atom, and a bromine atom.

Examples of the alkylamino group represented by $-NR^{11}R^{12}$ (here, $R^{11}$ and $R^{12}$ each independently represent a hydrogen atom; an alkyl group having 1 to 10 carbon atoms; or a cycloalkyl group having 3 to 10 carbon atoms) include a monoalkyl amino group or a dialkylamino group such as an N-methylamino group, an N,N-dimethylamino group, an N,N-diisopropylamino group, or an N-cyclohexylamino group. Examples of the 5-membered or 6-membered cyclic amino group include an unsaturated or saturated heterocyclic group having 5-membered to 6-membered 1 or 2 nitrogen atoms such as a pyrrolidinyl group, a piperidino group, or a morphonyl group.

Examples of the acyl group represented by $-CO-R^{11}$ (here, $R^{11}$ represents a hydrogen atom; an alkyl group having 1 to 10 carbon atoms; or a cycloalkyl group having 3 to 10 carbon atoms) include a formyl group, an acetyl group, a propionyl group, a butyryl group, a pivaloyl group, a pentanoyl group, a hexanoyl group, and the like.

Examples of the acylamino group represented by $-NH-CO-R^{11}$ (here, $R^{11}$ represents a hydrogen atom; an alkyl group having 1 to 10 carbon atoms; or a cycloalkyl group having 3 to 10 carbon atoms) include a formylamino group, an acetylamino group, a propionylamino group, a pivaloylamino group, a pentanoylamino group, a hexanoylamino group, and the like.

Examples of the alkoxy group represented by $-O-R^{11}$ (here, $R^{11}$ represents a hydrogen atom; an alkyl group having 1 to 10 carbon atoms; or a cycloalkyl group having 3 to 10 carbon atoms) include a methoxy group, an ethoxy group, an n-propoxy group, an isopropoxy group, an n-butoxy group, an s-butoxy group, an isobutoxy group, a t-butoxy group, an n-pentyloxy group, a 2-methylbutoxy group, a 3-methylbutoxy group, a 2,2-dimethylpropyloxy group, an n-hexyloxy group, a 2-methylpentyloxy group, a 3-methylpentyloxy group, a 4-methylpentyloxy group, a 5-methylpentyloxy group, a cyclohexyloxy group, and the like.

Examples of the alkoxy carbonyl group represented by $-COO-R^{11}$ (here, $R^{11}$ represents a hydrogen atom; an alkyl group having 1 to 10 carbon atoms; or a cycloalkyl group having 3 to 10 carbon atoms) include a methoxycarbonyl group, an ethoxycarbonyl group, an n-propoxycarbonyl group, an isopropoxycarbonyl group, an n-butoxycarbonyl group, a t-butoxycarbonyl group, a pentyloxycarbonyl group, a hexyloxycarbonyl group, a 2-ethylhexyloxycarbonyl group, and the like.

Examples of the alkylthio group represented by $-SR^{11}$ (here, $R^{11}$ represents a hydrogen atom, an alkyl group having 1 to 10 carbon atoms; or a cycloalkyl group having 3 to 10 carbon atoms) include a methylthio group, an ethylthio group, an n-propylthio group, an isopropylthio group, an n-butylthio group, an s-butylthio group, an isobutylthio group, a t-butylthio group, a pentylthio group, a hexylthio group, a cyclohexyl group, and the like.

Specific examples of the aryl group which may have a substituent, represented by $R^1$ of the general formulas (1-1) and (1-2), include a phenyl group, an o-, m-, and p-tolyl group, an o-, m-, and p-ethylphenyl group, an o-, m-, and p-isopropylphenyl group, an o-, m-, and p-t-butylphenyl group, a 2,4,6-trimethylphenyl group, a 2,4,6-triisopropylphenyl group, a 4-trifluoromethylphenyl group, a 2,4,6-trichlorophenyl group, a pentafluorophenyl group, and the like.

«Sulfonyl Moiety ($R^1$): Aralkyl group (which may have a substituent)»

Examples of the aralkyl group represented by $R^1$ of the general formulas (1-1) and (1-2) include a benzyl group, a phenethyl group, and the like.

Further, the aralkyl group represented by $R^1$ of the general formulas (1-1) and (1-2) may have an alkyl group

having 1 to 10 carbon atoms as a substituent.

Specific examples of the aralkyl group substituted with a substituent represented by $R^1$ of the general formulas (1-1) and (1-2) include an o-, m-, and p-methylbenzyl group, a 2,6-dimethylbenzyl group, a 2,4,6-trimethylbenzyl group, a 2,4,6-triisopropylbenzyl group, and the like.

<Diamine Moiety ($R^2$, $R^3$)>

$R^2$ and $R^3$ of the general formulas (1-1) and (1-2) each independently represent a hydrogen atom; or a phenyl group, or alternatively, $R^2$ and $R^3$ are bonded to each other to form a 4- to 8-membered cycloalkane ring with the carbon atom to which $R^2$ and $R^3$ are bonded.

«Diamine Moiety ($R^2$, $R^3$): Phenyl group»

The phenyl group represented by $R^2$ and $R^3$ of the general formulas (1-1) and (1-2) may have one or more substituents selected from an alkyl group, a halogen atom, and an alkoxy group.

The alkyl group as a substituent can be selected from the groups defined as the alkyl groups represented by $R^2$ and $R^3$ of the general formulas (1-1) and (1-2) described above, and preferably includes a linear or branched alkyl group having 1 to 5 carbon atoms.

Examples of the halogen atom as a substituent include a fluorine atom, a chlorine atom, and the like.

Examples of the alkoxy group as a substituent include a linear or branched alkoxy group having 1 to 10 carbon atoms, preferably 1 to 5 carbon atoms. Specific examples of the alkoxy group include a methoxy group, an ethoxy group, an n-propoxy group, an isopropoxy group, an n-butoxy group, an s-butoxy group, an isobutoxy group, a t-butoxy group, an n-pentyloxy group, an n-hexyloxy group, an n-heptyloxy group, an n-octyloxy group, an n-nonyloxy group, an n-decyloxy group, and the like.

«Diamine Moiety ($R^2$, $R^3$): $R^2$ and $R^3$ form a ring»

When $R^2$ and $R^3$ of the general formulas (1-1) and (1-2) are bonded to each other, $R^2$ and $R^3$ together with the carbon atom to which they are bonded form a linear or branched alkylene group having 2 to 10 carbon atoms, preferably 3 to 10 carbon atoms, forming a 4- to 8-membered ring, preferably a 5- to 8-membered cycloalkane ring, with adjacent carbon atoms. These rings may have an alkyl group such as a methyl group, an isopropyl group, or a t-butyl group as a substituent.

<Substituent on Amine ($R^4$)>

R4 in the general formulas (1-1) and (1-2) may represent a hydrogen atom; or an alkyl group which may have a substituent, or may be bonded to $R^5$ to form a cross-linking site of a divalent group represented by the formula (W). When forming the cross-linking site, the ruthenium complex is a tether type.

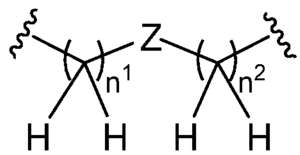 (W)

In the formula (W), a wavy line section at a carbon chain terminal containing $n^1$ is bonded to a carbon atom of an arene moiety instead of $R^5$ in the formulas (1-1) and (1-2), and a wavy line section at a carbon chain terminal containing $n^2$ in the formula (W) is bonded to a nitrogen atom of an amine moiety instead of $R^4$ in the formulas (1-1) and (1-2). Z represents a methylene group, an oxygen atom, or a sulfur atom. $n^1$ is an integer of 1 or 2, and $n^2$ is of 1, 2, or 3.

«Substituent on Amine ($R^4$): Alkyl group»

The alkyl group represented by $R^4$ of the general formulas (1-1) and (1-2) includes a linear or branched alkyl group having 1 to 10 carbon atoms, preferably 1 to 5 carbon atoms. Specific examples of the alkyl group include a methyl group, an ethyl group, an n-propyl group, an isopropyl group, an n-butyl group, an isobutyl group, an s-butyl group, a t-butyl group, an n-pentyl group, an n-hexyl group, an n-heptyl group, an n-octyl group, an n-nonyl group, an n-decyl group, and the like.

Further, the alkyl group represented by $R^4$ of the general formulas (1-1) and (1-2) may have one or more

substituents selected from halogen atoms such as a fluorine atom, a chlorine atom, and a bromine atom. Examples thereof include a perfluoroalkyl group such as a trifluoromethyl group, a pentafluoroethyl group, or a heptafluoropropyl group.

«Substituent on Amine ($R^4$): At the time of formation of a cross-linking site»

When $R^4$ of the general formulas (1-1) and (1-2) is bonded to $R^5$ to form a cross-linking site of a divalent group represented by the formula (W), Z in the general formula (W) represents a methylene group or an oxygen atom. Preferred Z includes an oxygen atom.

In the general formula (W), $n^1$ represents an integer of 1 or 2, and 1 is preferable as $n^1$. $n^2$ represents an integer of 1, 2, or 3, and 2 is preferable as $n^2$.

<Arene Moiety ($R^5$-$R^{10}$)>

$R^5$, $R^6$, $R^7$, $R^8$, $R^9$, and $R^{10}$ of the arene moiety represented by the general formulas (1-1) and (1-2) each independently represent a hydrogen atom; an alkyl group; a hydroxyl group; or an alkoxy group. Alternatively, $R^5$ together with $R^4$ may form a cross-linking site represented by the above formula (W).

«Arene Moiety ($R^5$-$R^{10}$): Alkyl group»

The alkyl groups represented by $R^5$, $R^6$, $R^7$, $R^8$, $R^9$, and $R^{10}$ of the general formulas (1-1) and (1-2) include a linear or branched alkyl group having 1 to 10 carbon atoms, preferably 1 to 5 carbon atoms. Specific examples of alkyl groups include a methyl group, an ethyl group, an n-propyl group, an isopropyl group, an n-butyl group, an isobutyl group, an s-butyl group, a t-butyl group, an n-pentyl group, an n-hexyl group, an n-heptyl group, an n-octyl group, an n-nonyl group, an n-decyl group, and the like.

«Arene Moiety ($R^5$-$R^{10}$): Alkoxy group»

The alkoxy groups represented by $R^5$, $R^6$, $R^7$, $R^8$, $R^9$, and $R^{10}$ of the general formulas (1-1) and (1-2) include a linear or branched alkoxy group having 1 to 10 carbon atoms, preferably 1 to 5 carbon atoms. Specific examples of the alkoxy groups include a methoxy group, an ethoxy group, an n-propoxy group, an isopropoxy group, an n-butoxy group, an s-butoxy group, an isobutoxy group, a t-butoxy group, an n-pentyloxy group, an n-hexyloxy group, an n-heptyloxy group, an n-octyloxy group, an n-nonyloxy group, an n-decyloxy group, and the like.

In the case of a complex in which $R^4$ and $R^5$ in the general formulas (1-1) and (1-2) do not form the cross-linking site represented by the formula (W) (that is, non-tether type ruthenium complex), $R^4$ is preferably a hydrogen atom, and/or $R^1$ is preferably a 4-methylphenyl group, a 2,3,4,5,6-pentafluorophenyl group, a methyl group, an isobutyl group, a benzyl group, a 2',5'-dimethylbenzyl group, or a 10-camphalyl group, and/or $R^2$ and $R^3$ are preferably phenyl groups or bonded to each other to form a cyclohexane ring, and/or $R^5$, $R^6$, $R^7$, $R^8$, $R^9$ and $R^{10}$ are preferably hydrogen atoms, methyl groups, or isopropyl groups.

Specific examples of the complex in which $R^4$ and $R^5$ in the general formulas (1-1) and (1-2) do not form the cross-linking site represented by the formula (W) (that is, non-tether type ruthenium complex) include, but are not limited to, the following compounds.

Note that hereinafter, the compound name and the abbreviation (in parenthesis) in the present specification are described together.

chloro(benzene[(R,R)-N-(p-toluenesulfonyl)-1,2-diphenylethylenediamine]-ruthenium(II)
(RuCl((R,R)-Ts-DPEN) (benzene))
chloro(benzene[(S,S)-N-(p-toluenesulfonyl)-1,2-diphenylethylenediamine]-ruthenium(II)
(RuCl((S,S)-Ts-DPEN) (benzene))
chloro(mesitylene)[(R,R)-N-(p-toluenesulfonyl)-1,2-diphenylethylenediamine]-ruthenium(II)
(RuCl((R,R)-Ts-DPEN) (mesitylene))
chloro(mesitylene)[(S,S)-N-(p-toluenesulfonyl)-1,2-diphenylethylenediamine]-ruthenium(II)
(RuCl((S,S)-Ts-DPEN) (mesitylene))
chloro(mesitylene)[(R,R)-N-(methanesulfonyl)-1,2-diphenylethylenediamine]-ruthenium(II)
(RuCl((R,R)-Ms-DPEN) (mesitylene))
chloro(mesitylene)[(S,S)-N-(methanesulfonyl)-1,2-diphenylethylenediamine]-ruthenium(II)
(RuCl((S, S)-Ms-DPEN) (mesitylene))
chloro(mesitylene)[(R,R)-N-(benzylsulfonyl)-1,2-diphenylethylenediamine]-ruthenium(II)

(RuCl((R,R)-$BnSO_2$-DPEN) (mesitylene))
chloro(mesitylene)[(S,S)-N-(benzylsulfonyl)-1,2-diphenylethylenediamine]-ruthenium(II)
(RuCl((S,S)-$BnSO_2$-DPEN) (mesitylene))
chloro(mesitylene)[(R,R)-N-(isobutanesulfonyl)-1,2-diphenylethylenediamine]-ruthenium(II)
(RuCl((R,R)-i-$BuSO_2$-DPEN) (mesitylene))
chloro(mesitylene)[(S,S)-N-(isobutanesulfonyl)-1,2-diphenylethylenediamine]-ruthenium(II)
(RuCl((S,S)-i-$BuSO_2$-DPEN) (mesitylene))
chloro(mesitylene)[(R,R)-N-(2',6'-dimethyl benzylsulfonyl)-1,2-diphenylethylenediamine]ruthenium (II)
(RuCl((R,R)-2',6',$(CH_3)_2BnSO_2$-DPEN) (mesitylene))
chloro(mesitylene)[(S,S)-N-(2',6'-dimethyl benzylsulfonyl)-1,2-diphenyl ethyl enediamine]ruthenium (II)
(RuCl((S,S)-2',6'-$(CH_3)_2BnSO_2$-DPEN) (mesitylene))
chloro(p-cymene)[(R,R)-N-(p-toluenesulfonyl)-1,2-diphenylethylenediamine]-ruthenium(II)
(RuCl((R,R)-Ts-DPEN) (p-cymene))
chloro(p-cymene)[(S,S)-N-(p-toluenesulfonyl)-1,2-diphenylethylenediamine]-ruthenium(II)
(RuCl((S,S)-Ts-DPEN) (p-cymene))
(p-cymene)[(R,R)-N-(p-toluenesulfonyl)-1,2-diphenylethylenediamine]-ruthenium(II)triflate
(Ru(OTf) ((R,R)-Ts-DPEN) (p-cymene))
(p-cymene)[(S,S)-N-(p-toluenesulfonyl)-1,2-diphenylethylenediamine]-ruthenium(II)triflate
(Ru(OTf) ((S,S)-Ts-DPEN) (p-cymene))
(p-cymene)[(R,R)-N-(p-toluenesulfonyl)-1,2-diphenylethylenediamine]-ruthenium(II)hydride
(RuH((R,R)-Ts-DPEN) (p-cymene))
(p-cymene)[(S,S)-N-(p-toluenesulfonyl)-1,2-diphenylethylenediamine]-ruthenium(II)hydride
(RuH((S,S)-Ts-DPEN) (p-cymene))
(p-cymene)[(R,R)-N-(p-toluenesulfonyl)-1,2-diphenylethylenediamine]-ruthenium(II)
(Ru((R,R)-Ts-DPEN) (p-cymene))
(p-cymene)[(S,S)-N-(p-toluenesulfonyl)-1,2-diphenylethylenediamine]- ruthenium(II) (Ru((S,S)-Ts-DPEN) (p-cymene))
chloro(p-cymene)[(R,R)-N-(methanesulfonyl)-1,2-diphenylethylenediamine]-ruthenium(II)
(RuCl((R,R)-Ms-DPEN) (p-cymene))
chloro(p-cymene)[(S,S)-N-(methanesulfonyl)-1,2-diphenylethylenediamine]-ruthenium(II)
(RuCl((S,S)-Ms-DPEN) (p-cymene))
chloro(p-cymene)[(R,R)-N-(pentafluorobenzenesulfonyl)-1,2-diphenylethylene-diamine]ruthenium(II)
(RuCl((R,R)-Fs-DPEN) (p-cymene))
chloro(p-cymene)[(S,S)-N-(pentafluorobenzenesulfonyl)-1,2-diphenylethylene-diamine]ruthenium(II)
(RuCl((S,S)-Fs-DPEN) (p-cymene))
chloro(p-cymene)[(R,R)-N-(benzylsulfonyl)-1,2-diphenylethylenediamine]-ruthenium(II)
(RuCl((R,R)-$BnSO_2$-DPEN) (p-cymene))
chloro(p-cymene)[(S,S)-N-(benzylsulfonyl)-1,2-diphenylethylenediamine]-ruthenium(II)
(RuCl((S,S)-$BnSO_2$-DPEN) (p-cymene))
chloro((p-cymene)[(R,R)-N-(isobutanesulfonyl)-1,2-diphenylethylenediamine]-ruthenium(II)
(RuCl((R,R)-i-$BuSO_2$-DPEN) (p-cymene))
chloro(p-cymene)[(S,S)-N-(isobutanesulfonyl)-1,2-diphenylethylenediamine]-ruthenium(II)
(RuCl((S,S)-i-$BuSO_2$-DPEN) (p-cymene))
chloro(p-cymene)[(R,R)-N-(2',6'-dimethyl benzylsulfonyl)-1,2-diphenyl ethyl enediamine]ruthenium (II)
(RuCl((R,R)-2',6',$(CH_3)_2BnSO_2$-DPEN) (p-cymene))
chloro(p-cymene)[(S,S)-N-(2',6'-dimethyl benzylsulfonyl)-1,2-diphenyl ethyl enediamine]ruthenium (II)
(RuCl((S,S)-2',6',$(CH_3)_2BnSO_2$-DPEN) (p-cymene))
chloro(p-cymene)[(R,R)-N-((1R)-camphorsulfonyl)-1,2-diphenylethylenediamine]-ruthenium(II)
(RuCl((R)-Cs-(R,R)-DPEN) (p-cymene))
chloro(p-cymene)[(S,S)-N-((1S)-camphorsulfonyl)-1,2-diphenylethylenediamine]-ruthenium(II)
(RuCl((S)-Cs-(S,S)-DPEN) (p-cymene))
chloro(p-cymene)[(R,R)-N-(p-toluenesulfonyl)-1,2-cyclohexanediamine]-ruthenium(II)
(RuCl((R,R)-Ts-DACH) (p-cymene))
chloro(p-cymene)[(S,S)-N-(p-toluenesulfonyl)-1,2-cyclohexanediamine]-ruthenium(II)
(RuCl((S,S)-Ts-DACH) (p-cymene))

In the case of a complex in which $R^4$ and $R^5$ in the general formulas (1-1) and (1-2) form the cross-linking site represented by the formula (W) (that is, tether type ruthenium complex), Z is preferably an oxygen atom, and/or $n^1$ is

preferably 1, and/or $n^2$ is preferably 2, and/or $R^1$ is a 4-methylphenyl group or a methyl group, and/or $R^2$ and $R^3$ are preferably phenyl groups, and/or $R^6$, $R^7$, $R^9$, and $R^{10}$ are preferably hydrogen atoms, and/or $R^8$ is preferably a methyl group.

Specific examples of the complex in which $R^4$ and $R^5$ in the general formulas (1-1) and (1-2) form the cross-linking site represented by the formula (W) (that is, tether type ruthenium complex) include, but are not limited to, the following compounds. Note that hereinafter, the compound name and the abbreviation (in parenthesis) in the present specification are described together.

chloro[(R,R)-N-[2-[2-(4-methylbenzyloxy)ethyl]amino-1,2-diphenylethyl]-p-toluenesulfonamide]ruthenium(II) ((R,R)-Ts-DENEB (registered trademark))
chloro[(S,S)-N-[2-[2-(4-methylbenzyloxy)ethyl]amino-1,2-diphenylethyl]-p-toluenesulfonamide]ruthenium(II) ((S,S)-Ts-DENEB (registered trademark))
chloro[(R,R)-N-[2-[2-(4-methylbenzyloxy)ethyl]amino-1,2-diphenylethyl]-methanesulfonamide]ruthenium(II) ((R,R)-Ms-DENEB (registered trademark))
chloro[(S,S)-N-[2-[2-(4-methylbenzyloxy)ethyl]amino-1,2-diphenylethyl]-methanesulfonamide]ruthenium(II) ((S,S)-Ms-DENEB (registered trademark))
chloro[(R,R)-N-[2-(3-phenylpropyl)amino-1,2-diphenylethyl]-p-toluenesulfonamide]ruthenium(II) (RuCl(benz-C3-teth-(R,R)-Ts-DPEN))
chloro[(S,S)-N-[2-(3-phenylpropyl)amino-1,2-diphenylethyl]-p-toluenesulfonamide]ruthenium(II) (RuCl(benz-C3-teth-(S,S)-Ts-DPEN))
chloro[(R,R)-N-[2-(3-phenylpropyl)amino-1,2-diphenylethyl]-methanesulfonamide]-ruthenium(II) (RuCl(benz-C3-teth-(R,R)-Ms-DPEN))
chloro[(S,S)-N-[2-(3-phenylpropyl)amino-1,2-diphenylethyl]-methanesulfonamide]-ruthenium(II) (RuCl(benz-C3-teth-(S,S)-Ms-DPEN))

The ruthenium complex, which is a constituent requirement in the catalyst according to the present invention, can be synthesized by those skilled in the art by, for example, the methods described in Japanese Patent No. 3040353 and Japanese Patent No. 5718178. Also, the ruthenium complex used may be a commercial one.

<Catalyst production method: Complex state and preparation solvent>

The catalyst of the present invention is obtained by reacting the ruthenium complex of the general formulas (1-1) and/or (1-2) with activated carbon in a solvent.

At the time of preparation, the state of the complex is not particularly limited, but it is desirable to dissolve it in a preparation solvent. The solvent used in this case is not particularly limited, but preferably a protonic polar solvent such as methanol, ethanol, or water, and particularly preferably methanol. If the complex is difficult to dissolve in the prepared solvent, two or more kinds of solvents may be mixed and used as needed.

Further, in the catalyst of the present invention, although not particularly limited, as described above, it is preferable to prepare the ruthenium complex so that the total mass thereof is usually in the range of 0.1% by mass to 25% by mass based on the total mass of the activated carbon.

«Supporting conditions: Heating and stirring conditions»

During the reaction, it may be stirred or allowed to stand. Heating may be performed or does not have to be performed during the preparation, and the reaction solution immediately after the start of preparation is generally dark brown, while decolorization of the reaction solution is observed over time with loading. The preparation end time varies depending on the type and the amount of the activated carbon and the complex, but in practice, various conditions may be set so that the loading is completed in 12 hours to 7 days. The prepared solvent may be removed by evaporation or by filtration.

«Supporting Conditions: On cleaning and drying conditions»

The activated carbon powder obtained after removing the preparation solvent is cleaned with an organic solvent. The cleaning solvent is not particularly limited, but it is preferable to use the same solvent as that used for preparation.

The obtained activated carbon support complex may contain or does not have to contain a cleaning solvent, but is preferably removed by drying under reduced pressure. The degree of vacuum during the drying under reduced pressure is not particularly limited, but is preferably about 20 torr or less. Heating may be performed if necessary, and the heating temperature is preferably 40°C or lower.

<Catalytic Reaction>

Using the catalyst of the present invention described above, and in the presence of a hydrogen donor, the organic compound can be reduced to produce a reduction product.

The hydrogenation of the present invention includes a method for producing an alcohol by reducing the carbonyl group of a carbonyl compound such as a ketone, and/or a method for producing an amine by reducing the imino group of an imine compound, using the catalyst according to the present invention and in the presence of a hydrogen donor. Further, when the above-mentioned activated carbon support ruthenium complex is an optically active substance, the carbonyl group of the carbonyl compound can be asymmetrically reduced to produce an optically active alcohol, and the imino group of the imine compound can be reduced to produce an optically active amine.

The hydrogen donor is not particularly limited as long as it is generally used for hydrogen transfer type hydrogenation, such as formic acid or an alkali metal salt thereof, or isopropanol which is an alcohol having a hydrogen atom at the $\alpha$-position of the carbon atom substituted by the hydroxyl group. Further, hydrogen gas can also be used as the hydrogen donor.

The solvent used in the reaction can be a hydrogen donor if it is a liquid, non-hydrogen donor solvents such as toluene, tetrahydrofuran, acetonitrile, dimethylformamide, dimethyl sulfoxide, acetone, and methylene chloride, water, methanol, ethanol, and n-butanol can be used alone or in combination.

«When Using Formate»

When a formate is used as a hydrogen donor in carrying out the hydrogenation, it is preferable to use water in combination with an organic solvent to dissolve the hydrogen donor. The organic solvent used is not particularly limited, but a solvent that is easily miscible with water is preferable, and examples thereof include methanol, ethanol, and n-butanol.

«When Using Formic Acid»

In carrying out the hydrogenation, when formic acid is used as the hydrogen donor, it is preferably carried out in the presence of a base such as a tertiary organic amine or an inorganic base, and more preferably carried out in the presence of a tertiary organic amine. The amine used includes, but not limited to, tertiary organic amines such as trimethylamine, triethylamine, triisopropylamine, 1,4-diazabicyclo[2,2,2]octane (DABCO), and 1,8-diazabicyclo[5,4,0]undec-7-ene (DBU).

In this case, formic acid and amine may be added to the reaction system separately, or may be mixed and added to the reaction system. When used as a mixture, formic acid and amine can be mixed and used in any ratio. Further, an azeotropic mixture of formic acid and amine may be prepared and used in advance. Examples of preferable formic acid and amine azeotropic mixtures include triethylamine formate (5:2 (molar ratio)) azeotropic mixtures.

When the catalyst according to the present invention is used as a hydrogenation catalyst, the amount used is preferably selected from the range in which the molar ratio (S/C) of the substrate (carbonyl compound or imines) (S) to the ruthenium metal atoms (C) is 10 to 1000000, preferably 50 to 15000.

«Amount of Hydrogen Donor»

The amount of the hydrogen donor to the carbonyl compound is usually equal to or more than an equimolar amount, and when the hydrogen donor is formic acid or a salt thereof, it is preferably used in a range of 1.5 times molar amount or more, and 40 times molar amount or less, preferably 20 times molar amount or less, and is preferably 2 to 20 times the molar amount from the viewpoint of molecular efficiency. On the other hand, when the hydrogen donor is isopropanol or the like, it is used in a large excess with respect to the substrate from the viewpoint of reaction equilibrium, and is usually used in the range of 100 times molar amount or less.

«Details of Reaction Conditions»

The reaction temperature is selected from the range of -20 to 100°C, preferably 0 to 70°C. The reaction pressure is not particularly limited, and is usually carried out under 0.5 to 2 atm, preferably normal pressure. When hydrogen gas is used, it is usually 5 MPa or less.

The reaction time varies depending on the catalytic ratio to the substrate, but is 1 to 100 hours, usually 2 to 50 hours.

<<On Reuse>>

After the reaction is completed, the catalyst according to the present invention and the reaction solution with the reduced target substance can be separated by a simple method such as decantation or filtration. Further, the catalyst of the present invention separated by decantation or the like can be reused as it is for the next reaction. From the reaction solution after removing the catalyst, the produced products or optically active substances can be separated and purified by general operations such as distillation, extraction, chromatography, and recrystallization.

<<On Flow Reaction>>

Further, in the hydrogenation of the present invention, a continuous flow reaction can be carried out by taking advantage of the features of the activated carbon support ruthenium complex. That is, after the column is filled with the activated carbon support ruthenium complex, the substrate, the hydrogen donor, and the solvent are pumped by a metering pump while keeping the column at the above reaction temperature, and this makes it possible to reduce the organic compound to produce a reduction product without the need for a catalyst separation operation. The reaction conditions are the same as above.

The reaction can be carried out, for example, by preparing a mixed solution of a substrate, a solvent, a base, and a hydrogen donor and feeding the solution to a column using an HPLC pump. When the hydrogen donor is hydrogen gas, the substrate, solvent, and base can be mixed, the mixed solution can be fed to the column, and the hydrogen gas can be passed through the column for reaction.

[Examples]

Hereinafter, the compound of the present invention and the catalytic reaction using the catalyst of the present invention will be described in detail with reference to Examples, but the present invention is not limited to these Examples. In the examples, the devices and conditions used for measuring the physical properties are as follows.

1) Gas chromatography (GC): GC-4000 Plus type device (manufactured by GL Sciences)

[Measurement Condition 1] Column: CP-Chirasil-DEX CB (manufactured by Agilent), sample introduction unit: 250°C, sample detection unit: 250°C, measurement temperature: 120°C, retention time: 15 minutes.
[Measurement Condition 2] Column: CP-Chirasil-DEX CB (manufactured by Agilent), sample introduction unit: 250°C, sample detection unit: 250°C, measurement temperature: 120°C, retention time: 30 minutes.
[Measurement Condition 3] Column: CP-Chirasil-DEX CB (manufactured by Agilent), sample introduction unit: 250°C, sample detection unit: 250°C, measurement temperature: 150°C, retention time: 25 minutes.
[Measurement Condition 4] Column: CP-Chirasil-DEX CB (manufactured by Agilent), sample introduction unit: 250°C, sample detection unit: 250°C, measurement temperature: 120°C, retention time: 35 minutes.
[Measurement Condition 5] Column: CP-Chirasil-DEX CB (manufactured by Agilent), sample introduction unit: 250°C, sample detection unit: 250°C, measurement temperature: 60°C, retention time: 30 minutes.

2) Plasma (ICP) optical emission spectroscopic analyzer

ICP optical emission spectroscopy was used to analyze the amount of metal eluted. In the Examples, the apparatus and conditions used for the analysis of the amount of ruthenium eluted are as follows.
Apparatus: Optima 8300 (manufactured by Perkin Elmer)
High frequency output: 1.5 kW
Plasma gas flow rate: 12 mL/min (argon gas)
Auxiliary gas flow rate: 0.4 L/min (argon gas)
Carrier gas flow rate: 0.35 L/min (argon gas)
Sample introduction rate: 0.8 mL/min
Chamber: Cyclone chamber

3) Proton nuclear magnetic resonance spectroscopy ($^1H$ NMR): 400-MR DD2 type device (resonance frequency: 400 MHz) (manufactured by Agilent)
4) SEM-EDS
SEM-EDS was used for the analysis of ruthenium on the catalyst.

Analyzer: TM-3000 (manufactured by Hitachi High-Tech Science Corporation)

Analysis software: SwiftED Ver. 1.7 (manufactured by Oxford Instruments)
Acceleration voltage: 15 kV

Table 1 shows the characteristic X-ray energies of the elements analyzed.

Table 1

| Element | Characteristic X-Ray Energy (kV) | Element | Characteristic X-Ray Energy (kV) | Element | Characteristic X-Ray Energy (kV) |
|---|---|---|---|---|---|
| C | 0.28 | Cl | 2.62 | Zn | 8.64 |
| N | 0.39 | | 2.82 | | 9.57 |
| O | 0.53 | | 0.18 | | 1.01 |
| Na | 1.04 | K | 3.31 | | 0.71 |
| Mg | 1.25 | | 3.59 | | 0.88 |
| Al | 1.49 | | 0.26 | Ru | 19.28 |
| Si | 1.74 | Ca | 3.69 | | 2.56 |
| P | 2.01 | | 4.01 | | 2.68 |
| | 2.14 | | 0.34 | | 2.38 |
| S | 2.31 | | 0.31 | | 3.18 |
| | 2.46 | | | | 2.25 |

Table 2 shows the activated carbon used in the present Examples.

Table 2

| | Residue on Ignition (%) | Specific Surface Area ($m^2/g$) | Avg. Pore Diameter (nm) | Avg. Particle Diameter (μm) | Surface Functional Group (mmol/g) |
|---|---|---|---|---|---|
| A1 | - | 1583 | 1.84 | - | - |
| A2 | 0.2 | 1610 | 1.84 | 44.7 | 0.15 |
| B1 | - | 927 | 1.82 | - | - |
| B2 | - | 994 | 1.81 | - | - |
| C1 | - | 1455 | 3.57 | - | - |
| M | - | 899 | 2.23 | - | - |
| C2 | 0.3 | 1467 | 3.84 | 57.1 | 0.23 |
| A1, Granular | - | 1685 | 1.85 | - | - |
| A2, Granular | 0.1 | 1739 | 1.86 | 324 | 0.16 |
| B1, Granular | - | 1044 | 1.77 | - | - |
| B2, Granular | - | 1037 | 1.76 | - | - |
| C2, Granular | 0.6 | 1487 | 3.29 | 380 | 0.22 |

Note that as described above, "DENEB" described in the Examples is a registered trademark of Takasago International Corporation.

[Example 1] Preparation of a catalyst (catalyst 1) containing chloro[(R,R)-N-[2-[2-(4-methylbenzyloxy)ethyl]amino-1,2-diphenylethyl]-p-toluenesulfonamide]ruthenium(II) adsorbed on powdery activated carbon (A1)

To a 200 mL beaker, chloro[(R,R)-N-[2-[2-(4-methylbenzyloxy)ethyl]amino-1,2-diphenylethyl]-p-toluenesulfonamide]ruthenium(II) ((R,R)-Ts-DENEB (manufactured by Takasago International Corporation), 40.7 mg, 0.0626 mmol), dehydrated toluene (39 mL), and dehydrated ethanol (2 mL) were added and completely dissolved to prepare a complex solution. Activated carbon (A1, 5.00 g) was added to a 300 mL beaker, and then the complex solution was added over 1 minute. The resulting suspension was allowed to stand until the solvent was evaporated. Dehydrated toluene (30 mL) and dehydrated ethanol (1.5 mL) were added to the resulting solid components, and the mixture was stirred for 1 minute and then filtered. The resulting solid was transferred to a 300 mL beaker, and then dehydrated toluene (30 mL) and dehydrated ethanol (1.5 mL) were added thereto, and the mixture was stirred for 1 minute and then filtered. The solid components recovered by filtration were dried under reduced pressure at 40°C for 24 hours to obtain 6.56 g of compound 1. The filtrate was concentrated under reduced pressure, and the amount of ruthenium contained in the recovered residue was measured by ICP analysis. As a result, the unadsorbed ruthenium components were 0.3% based on ruthenium components used. From this, it was found that the adsorption rate of the complex was 99.7%, and the loading amount as the complex to the activated carbon was 0.81% by mass.

[Example 2] Preparation of a catalyst (catalyst 2) containing a (R,R)-Ts-DENEB complex adsorbed on powdery activated carbon (A2)

Compound 2 in an amount of 6.62 g was obtained by the same method as in Example 1 except that A2 (5.00 g) was used as the activated carbon. The filtrate was concentrated under reduced pressure, and the amount of ruthenium contained in the recovered trace amount of residue was measured by ICP analysis. As a result, the unadsorbed ruthenium components were 0.7%. From this, it was found that the adsorption rate of the complex was 99.3%, and the loading amount as the complex to the activated carbon was 0.80% by mass.

[Example 3] Preparation of a catalyst (catalyst 3) containing a (R,R)-Ts-DENEB complex adsorbed on powdery activated carbon (B1)

Compound 3 in an amount of 6.10 g was obtained by the same method as in Example 1 except that B1 (5.00 g) was used as the activated carbon. When the filtrate was concentrated under reduced pressure, an unadsorbed complex was recovered as a solid phase component. The mass of the recovered complex was 8.9 mg. From this, the adsorption rate of the complex was 78.1%, and the loading amount as the complex to the activated carbon was 0.63% by mass.

[Example 4] Preparation of a catalyst (catalyst 4) containing a (R,R)-Ts-DENEB complex adsorbed on powdery activated carbon (B2)

Compound 4 in an amount of 6.17 g was obtained by the same method as in Example 1 except that B2 (5.00 g) was used as the activated carbon. When the filtrate was concentrated under reduced pressure, an unadsorbed complex was recovered as a solid phase component. The mass of the recovered complex was 9.0 mg. From this, the adsorption rate of the complex was 77.9%, and the loading amount as the complex to the activated carbon was 0.63% by mass.

[Example 5] Preparation of a catalyst (catalyst 5) containing a (R,R)-Ts-DENEB complex adsorbed on powdery activated carbon (C1)

Compound 5 in an amount of 5.74 g was obtained by the same method as in Example 1 except that C1 (5.00 g) was used as the activated carbon. The filtrate was concentrated under reduced pressure, but no solid phase components were recovered. That is, the adsorption rate of the complex was 100%, and the loading amount as the complex to the activated carbon was 0.81% by mass.

[Example 6] Preparation of a catalyst (catalyst 6) containing a (R,R)-Ts-DENEB complex adsorbed on powdery activated carbon (C2)

Compound 6 in an amount of 5.85 g was obtained by the same method as in Example 1 except that C2 (5.00 g) was used as the activated carbon. The filtrate was concentrated under reduced pressure, and the amount of ruthenium contained in the recovered trace amount of residue was measured by ICP analysis. As a result, the unadsorbed ruthenium

components were 1.0%. From this, it was found that the adsorption rate of the complex was 99.0%, and the loading amount as the complex to the activated carbon was 0.80% by mass.

[Example 7] Preparation of a catalyst (catalyst 7) containing a (R,R)-Ts-DENEB complex adsorbed on powdery activated carbon (M)

Compound 7 in an amount of 5.82 g was obtained by the same method as in Example 1 except that M (5.00 g) was used as the activated carbon. The filtrate was concentrated under reduced pressure, but no solid phase components were recovered. That is, the adsorption rate of the complex was 100%, and the loading amount as the complex to the activated carbon was 0.81% by mass.

[Example 8] Preparation of a catalyst (catalyst 8) containing a (R,R)-Ts-DENEB complex adsorbed on granular activated carbon (A1, granular)

Compound 8 in an amount of 6.02 g was obtained by the same method as in Example 1 except that A1 (granular, 5.00 g) was used as the activated carbon. The filtrate was concentrated under reduced pressure, but no solid phase components were recovered. That is, the adsorption rate of the complex was 100%, and the loading amount as the complex to the activated carbon was 0.81% by mass.

[Example 9] Preparation of a catalyst (catalyst 9) containing a (R,R)-Ts-DENEB complex adsorbed on granular activated carbon (A2, granular)

Compound 9 in an amount of 6.56 g was obtained by the same method as in Example 1 except that A2 (granular, 5.00 g) was used as the activated carbon. The filtrate was concentrated under reduced pressure, but no solid phase components were recovered. That is, the adsorption rate of the complex was 100%, and the loading amount as the complex to the activated carbon was 0.81% by mass.

[Example 10] Preparation of a catalyst (catalyst 10) containing a (R,R)-Ts-DENEB complex adsorbed on granular activated carbon (B1 granules)

Compound 10 in an amount of 5.84 g was obtained by the same method as in Example 1 except that B1 (granular, 5.00 g) was used as the activated carbon. When the filtrate was concentrated under reduced pressure, an unadsorbed complex was recovered as a solid phase component. The mass of the recovered complex was 12.0 mg. From this, the adsorption rate of the complex was 70.5%, and the loading amount as the complex to the activated carbon was 0.57% by mass.

[Example 11] Preparation of a catalyst (catalyst 11) containing a (R,R)-Ts-DENEB complex adsorbed on granular activated carbon (B2, granular)

Compound 11 in an amount of 6.01 g was obtained by the same method as in Example 1 except that B2 (granular, 5.00 g) was used as the activated carbon. When the filtrate was concentrated under reduced pressure, an unadsorbed complex was recovered as a solid phase component. The mass of the recovered complex was 12.1 mg. From this, the adsorption rate of the complex was 70.3%, and the loading amount as the complex to the activated carbon was 0.57% by mass.

[Example 12] Preparation of a catalyst (catalyst 12) containing a (R,R)-Ts-DENEB complex adsorbed on granular activated carbon (C2, granular)

Compound 12 in an amount of 5.60 g was obtained by the same method as in Example 1 except that the activated carbon was changed to C2 (granular, 5.00 g). The filtrate was concentrated with an evaporator, but no solid phase components were recovered. That is, the adsorption rate of the complex was 100%, and the loading amount as the complex to the activated carbon was 0.81% by mass.

Table 3 shows the preparation results of catalysts including the Ts-DENEB catalysts adsorbed on various activated carbons.

Table 3: Preparation results of catalysts including the Ts-DENEB catalysts adsorbed on activated carbons

| Catalyst No. | Activated Carbon | Loading Amount as a Complex to Activated Carbon (Mass %) |
|---|---|---|
| 1 | A1 | 0.81 |
| 2 | A2 | 0.80 |
| 3 | B1 | 0.63 |
| 4 | B2 | 0.63 |
| 5 | C1 | 0.81 |
| 6 | C2 | 0.80 |
| 7 | M | 0.81 |
| 8 | A1, Granular | 0.81 |
| 9 | A2, Granular | 0.81 |
| 10 | B1, Granular | 0.57 |
| 11 | B2, Granular | 0.57 |
| 12 | C2, Granular | 0.81 |

In this way, it was possible to support a Ts-DENEB complex on various activated carbons.

[Example 13] Preparation of a catalyst (catalyst 13) containing a (R,R)-Ts-DENEB complex adsorbed on granular activated carbon (A2, granular)

(R,R)-Ts-DENEB (199.9 mg, 0.307 mmol) was charged in a 200 mL round-bottom flask with stopcock, and the inside of the apparatus was replaced with nitrogen. Then, dehydrated toluene (57 mL) and dehydrated ethanol (3 mL) were added to dissolve. Subsequently, activated carbon manufactured by Osaka Gas Chemicals Co., Ltd. (A2, granular, 4.96 g) was added, and the mixture was allowed to stand at room temperature for 1 week under a nitrogen atmosphere. After removing the supernatant by filtration under reduced pressure, the filtered solid was transferred to a 300 mL beaker. Dehydrated toluene (30 mL) and dehydrated ethanol (1.5 mL) were added, and the mixture was stirred for 1 minute and then filtered. The same operation was repeated twice, and the solid components recovered by filtration were dried under reduced pressure at 40°C for 24 hours to obtain 6.12 g of compound 13. When the filtrate was concentrated under reduced pressure, an unadsorbed complex was recovered as a solid phase component. The mass of the recovered complex was 107.0 mg. From this, the adsorption rate of the complex was 46.5%, and the loading amount as the complex to the activated carbon was 1.9% by mass.

[Example 14] Preparation of a catalyst (catalyst 14) containing a Ts-DENEB complex adsorbed on granular activated carbon (A2, granular)

Compound 14 in an amount of 6.20 g was obtained by the same method as in Example 13 except that the amount of Ts-DENEB charged was changed to 400.4 mg (0.616 mmol), the amount of activated carbon A2 (granular) charged was changed to 5.00 g, the amount of dehydrated toluene charged was changed to 114 mL, and the amount of dehydrated ethanol charged was changed to 6 mL. When the filtrate was concentrated with an evaporator, an unadsorbed complex was recovered as a solid phase component. The mass of the recovered complex was 278.5 mg. From this, the amount of the complex adsorbed on the activated carbon was 30.4%, and the loading amount as the complex to the activated carbon was 2.4% by mass.

[Example 15] Preparation of a catalyst (catalyst 15) containing a (R,R)-Ts-DENEB complex adsorbed on granular activated carbon (A2, granular)

(R,R)-Ts-DENEB (199.7 mg, 0.307 mmol) was charged in a 200 mL round-bottom flask with stopcock, and the inside of the apparatus was replaced with nitrogen. Then, dehydrated methanol (60 mL) was added to dissolve. Subsequently, activated carbon manufactured by Osaka Gas Chemicals Co., Ltd. (A2, granular, 4.87 g) was added, and the mixture was allowed to stand at room temperature for 1 week under a nitrogen atmosphere. After removing the

supernatant by filtration under reduced pressure, the filtered solid was transferred to a 300 mL beaker. Dehydrated methanol (30 mL) was added, and the mixture was stirred for 1 minute and then filtered. The same operation was repeated twice, and the solid components recovered by filtration were dried under reduced pressure at 40°C for 24 hours to obtain 5.03 g of compound 15. When the filtrate was concentrated under reduced pressure, an unadsorbed complex was recovered as a solid phase component. The mass of the recovered complex was 6.9 mg. From this, the amount of the complex adsorbed on the activated carbon based on the activated carbon was 96.5%, and the loading amount as the complex was 3.9% by mass.

[Example 16] Preparation of a catalyst (catalyst 16) containing a (R,R)-Ts-DENEB complex adsorbed on granular activated carbon (A2, granular)

Compound 16 in an amount of 5.12 g was obtained by the same method as in Example 15 except that the amount of (R,R)-Ts-DENEB charged was changed to 400.0 mg (0.615 mmol), the amount of activated carbon (A2, granular) charged was changed to 4.96 g, and the amount of dehydrated methanol charged was changed to 120 mL. When the filtrate was concentrated under reduced pressure, an unadsorbed complex was recovered as a solid phase component. The mass of the recovered complex was 34.2 mg. From this, the amount of the complex adsorbed on the activated carbon was 91.4%, and the loading amount as the complex to the activated carbon was 7.3% by mass.

[Example 17] Preparation of a catalyst (catalyst 17) containing a (R,R)-Ts-DENEB complex adsorbed on granular activated carbon (C2, granular)

Compound 17 was obtained by the same method as in Example 16 except that the activated carbon was changed to C2 (granular, 4.90 g). When the filtrate was concentrated under reduced pressure, an unadsorbed complex was recovered as a solid phase component. The mass of the recovered complex was 44.2 mg. From this, the amount of the complex adsorbed on the activated carbon was 89.0%, and the loading amount as the complex to the activated carbon was 7.2% by mass.

[Example 18] Preparation of a catalyst (catalyst 18) containing a (R,R)-Ts-DENEB complex adsorbed on granular activated carbon (A2, granular)

(R,R)-Ts-DENEB (400.0 mg, 0.0615 mmol) and dehydrated methanol (100 mL) were added to and dissolved in a 200 mL beaker to prepare a complex solution. Activated carbon (A2, granular, 5.00 g) was added to a 300 mL beaker, and then the complex solution was added over 1 minute. The resulting suspension was allowed to stand until the solvent was distilled off. Dehydrated methanol (30 mL) was added to the resulting solid components, and the mixture was stirred for 1 minute and then filtered. The same operation was repeated twice, and the solid components recovered by filtration were dried under reduced pressure at 40°C for 24 hours to obtain 5.10 g of compound 18. When the filtrate was concentrated under reduced pressure, an unadsorbed complex was recovered as a solid phase component. The mass of the recovered complex was 15.7 mg. From this, the adsorption rate of the complex was 96.1%, and the loading amount as the complex to the activated carbon was 7.7% by mass.

[Example 19] Preparation of a catalyst (catalyst 19) containing a (R,R)-Ts-DENEB complex adsorbed on granular activated carbon (C2, granular)

Compound 19 was obtained by the same method as in Example 18 except that the activated carbon was changed to C2 (granular, 5.00 g). When the filtrate was concentrated under reduced pressure, an unadsorbed complex was recovered as a solid phase component. The mass of the recovered complex was 15.1 mg. From this, the adsorption rate of the complex was 96.2%, and the loading amount as the complex to the activated carbon was 7.7% by mass.

[Example 20] Preparation of a catalyst (catalyst 20) containing a (R,R)-Ts-DENEB complex adsorbed on granular activated carbon (C2, granular)

Compound 20 was obtained by the same method as in Example 17 except that the amount of (R,R)-Ts-DENEB charged was changed to 1200.0 mg (1.846 mmol). When the filtrate was concentrated under reduced pressure, an unadsorbed complex was recovered as a solid phase component. The mass of the recovered complex was 109.4 mg. From this, the adsorption rate of the complex was 90.9%, and the loading amount as the complex to the activated carbon was 21.8% by mass.

Table 4 shows the preparation results of catalysts including the (R,R)-Ts-DENEB complex adsorbed on activated carbons obtained under various preparation conditions.

Table 4: Preparation results of catalysts including the (R,R)-Ts-DENEB complex adsorbed on activated carbons obtained under various preparation conditions

| Catalyst No. | Activated Carbon | Concentration of Solvent | Preparation Solvent | Charged Amount of a Complex to Activated Carbon (Mass %) | Loading Amount of a Complex to Activated Carbon (Mass %) |
|---|---|---|---|---|---|
| 13 | A2, Granular | N/A | Toluene Ethanol | 4.0 | 1.9 |
| 14 | A2, Granular | N/A | Toluene Ethanol | 8.0 | 2.4 |
| 15 | A2, Granular | N/A | Methanol | 4.0 | 3.9 |
| 16 | A2, Granular | N/A | Methanol | 8.0 | 7.3 |
| 18 | A2, Granular | Yes | Methanol | 8.0 | 7.7 |
| 17 | C2, Granular | N/A | Methanol | 8.0 | 7.2 |
| 19 | C2, Granular | Yes | Methanol | 8.0 | 7.7 |
| 20 | C2, Granular | N/A | Methanol | 24.0 | 21.8 |

As described above, by appropriately adjusting the type and concentration of the solvent, it was possible to increase the loading amount of the complex.

<Analysis 1> Photographing of ruthenium element mapping images of catalyst 17 and catalyst 19 by SEM-EDS

For catalyst 17 and catalyst 19, the integration time was set to 900 seconds, and SEM-EDS was used to obtain SEM images and element mapping images with ruthenium as the target element. Fig. 4 shows the results. From these element mapping images, it was confirmed that (R,R)-Ts-DENEB was widely dispersed on the activated carbon.

<Analysis 2> Calculation of the mass percentage of (R,R)-Ts-DENEB in activated carbon surface analysis of catalyst 17 and catalyst 19 by SEM-EDS

The surface analysis of catalyst 17 and catalyst 19 was measured by SEM-EDS. The selected elements to be analyzed were 9 kinds of elements expected to be contained in activated carbon, carbon, nitrogen, oxygen, sodium, magnesium, phosphorus, potassium, calcium, and zinc, and 3 kinds of elements contained in (R,R)-Ts-DENEB, sulfur, chlorine, and ruthenium, 12 kinds in total. The mass percentage of (R,R)-Ts-DENEB adsorbed on the activated carbon was calculated from the mass percentage of ruthenium on the surface of the activated carbon obtained by SEM-EDS. Table 5 shows the results.

Table 5

| | Catalyst 17 | Catalyst 19 |
|---|---|---|
| Mass Percentage of Ts-DENEB Calculated by Surface Analysis of Activated Carbon | 6.1 | 8.0 |

<Analysis 3> Calculation of the mass percentage of (R,R)-Ts-DENEB by elemental analysis of ash content of catalyst 17 and catalyst 19.

Catalyst 17 and catalyst 19 were each dried for 3 hours in an electric dryer adjusted to 115°C±5°C, and about 150 mg of a sample cooled in a desiccator (using silica gel as a desiccant) for 1 hour was weighed into a crucible. Then, the sample was put into an electric furnace heated to 850°C, and heated in an air atmosphere for 7 hours. After cooling

in the air, the weight of the ash content was measured, and then the obtained ash content was analyzed by SEM-EDS in the same manner as in analysis 2 (note that in this analysis, the integration time was set to 300 seconds, and a total of 14 types were targeted, the 12 types of elements described in analysis 2 as well as 2 types, aluminum and silicon, as elements that could be mixed from the crucible). The mass percentage of (R,R)-Ts-DENEB adsorbed on the activated carbon was determined by the following formula. Table 6 shows the results.

$$W_{cat} = \frac{R \times w_{Ru} \times \frac{M}{101.07}}{S}$$

($W_{cat}$: Mass percentage of (R,R)-Ts-DENEB adsorbed on the activated carbon
S: Mass of the activated carbon (g)
R: Mass of the residue (g)
$W_{Ru}$: Mass percentage of ruthenium obtained by SEM-EDS
M: Molecular weight of (R,R)-Ts-DENEB)

Table 6

| | Catalyst 17 | Catalyst 19 |
|---|---|---|
| Ash Content (mg) | 2.3 | 3.3 |
| $W_{cat}$ (Mass %) | 6.4 | 8.1 |

It was found that the mass percentage of (R,R)-Ts-DENEB calculated from the surface composition of the activated carbon in analysis 2 showed a good agreement with the value calculated from the ash composition in analysis 3. From these results, it was inferred that (R,R)-Ts-DENEB was uniformly supported on the surface of the activated carbon and inside thereof.

[Example 21] Preparation of a catalyst (catalyst 21) containing a chloro[(R,R)-N-[2-[2-(4-methylbenzyloxy)ethyl]amino-1,2-diphenylethyl]-p-methanesulfonamide]ruthenium(II) complex adsorbed on granular activated carbon (C2, granular)

Chloro[(R,R)-N-[2-[2-(4-methylbenzyloxy)ethyl]amino-1,2-diphenylethyl]-p-methanesulfonamide]ruthenium(II) ((R,R)-Ms-DENEB, 142.2mg, 0.248 mmol) was charged in a 200 mL round-bottom flask with stopcock, and the inside of the apparatus was replaced with nitrogen. Then, dehydrated methanol (48 mL) was added to dissolve. Subsequently, activated carbon manufactured by Osaka Gas Chemicals Co., Ltd. (C2, granular, 2.00 g) was added, and the mixture was allowed to stand at room temperature for 1 week under a nitrogen atmosphere. After removing the supernatant by filtration under reduced pressure, the filtered solid was transferred to a 300 mL beaker. Dehydrated methanol (25 mL) was added, and the mixture was stirred for 1 minute and then filtered. The same operation was repeated twice, and the solid components recovered by filtration were dried under reduced pressure at 40°C for 24 hours to obtain compound 21. When the filtrate was concentrated under reduced pressure, an unadsorbed complex was recovered as a solid phase component. The mass of the recovered complex was 20.0 mg. From this, the amount of the complex adsorbed on the activated carbon was 85.9%, and the loading amount as the complex to the activated carbon was 6.1% by mass.

[Example 22] Preparation of a catalyst (catalyst 22) containing a chloro[(R,R)-N-[2-(3-phenylpropyl)amino-1,2-diphenylethyl]-p-toluenesulfonamide]ruthenium(II) complex adsorbed on granular activated carbon (C2, granular)

Compound 22 was obtained by the same method as in Example 21 except that the complex was changed to chloro[(R,R)-N-[2-(3-phenylpropyl)amino-1,2-diphenylethyl] - p-toluenesulfonamide]ruthenium(II) (RuCl(benz-C3-teth-(R,R)-Ts-DPEN), manufactured by STREM, 153.1 mg, 0.247 mmol). When the filtrate was concentrated under reduced pressure, an unadsorbed complex was recovered as a solid phase component. The mass of the recovered complex was 22.3 mg. From this, the amount of the complex adsorbed on the activated carbon was 85.4%, and the loading amount as the complex to the activated carbon was 6.5% by mass.

[Example 23] Preparation of a catalyst (catalyst 23) containing a chloro(p-cymene)[(R,R)-N-(p-toluenesulfonyl)-1,2-diphenylethylenediamine]ruthenium(II) adsorbed on granular activated carbon (C2, granular)

Compound 23 was obtained by the same method as in Example 21 except that the complex was changed to chloro(p-cymene)[(R,R)-N-(p-toluenesulfonyl)-1,2-diphenylethylenediamine]ruthenium(II) (RuCl((R,R)-Ts-DPEN) (p-cymene), manufactured by Takasago International Corporation), 157.8 mg, 0.248 mmol). When the filtrate was concentrated under reduced pressure, an unadsorbed complex was recovered as a solid phase component. The mass of the recovered complex was 33.9 mg. From this, the amount of the complex adsorbed on the activated carbon was 78.5%, and the loading amount as the complex to the activated carbon as a complex was 6.2% by mass.

[Example 24] Preparation of a catalyst (catalyst 24) containing a chloro(benzene)[(R,R)-N-(p-toluenesulfonyl)-1,2-diphenylethylenediamine]- ruthenium(II) complex adsorbed on granular activated carbon (C2, granular)

Compound 24 was obtained in the same manner as in Example 21 except that the complex was changed to chloro(benzene[(R,R)-N-(p-toluenesulfonyl)-1,2-diphenylethylenediamine]ruthenium(II) ((R,R)-RuCl(Ts-DPEN)(benzene), manufactured by Takasago International Corporation, 43.3 mg, 0.247 mmol). When the filtrate was concentrated under reduced pressure, an unadsorbed complex was recovered as a solid phase component. The mass of the recovered complex was 29.1 mg. From this, the amount of the complex adsorbed on the activated carbon was 79.7%, and the loading amount as the complex to the activated carbon was 5.7% by mass.

Table 7 shows the examination results of various complexes adsorbed on activated carbon.

Table 7: Results of support examination of various complexes adsorbed on activated carbon

| Catalyst No. | Complex | Loading Amount of a Complex to Activated Carbon (Mass %) |
|---|---|---|
| 21 | (R,R)-Ms-DENEB | 6.1 |
| 22 | RuCl(benz-C3-teth-(R,R)-Ts-DPEN) | 6.5 |
| 23 | RuCl((R,R)-Ts-DPEN) (p-cymene) | 6.2 |
| 24 | RuCl((R,R)-Ts-DPEN) (benzene) | 5.7 |

As described above, it was found that the support on activated carbon was not limited to Ts-DENEB and was effective for a wide range of ruthenium complexes having a diamine skeleton.

[Example 25] Production of (R)-1-phenylethyl alcohol by asymmetric transfer hydrogenation of acetophenone using a catalyst (catalyst 1) containing a (R,R)-Ts-DENEB complex adsorbed on activated carbon (A1)

(*R,R*)-Ts-DENEB/Activated Carbon (0.5 mol%)
Potassium Formate (20 eq.)

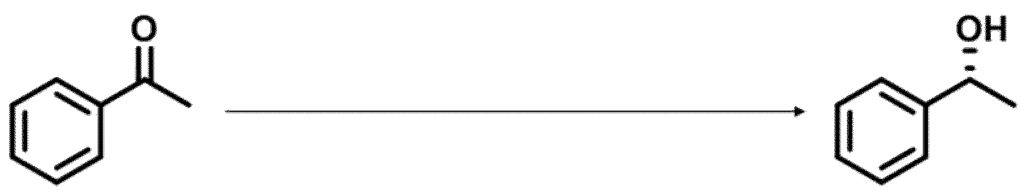

1mmol

Distilled Water (5 mL)
Dehydrated Ethanol (2 mL)
60°C, 2 Hours

A magnetic stirring bar, catalyst 1 prepared in Example 1 (0.43 g, 0.005 mmol, 0.5 mol%), and potassium formate (1.68 g, 20 mmol) were charged in an 80 mL Schlenk tube, and the inside of the apparatus was replaced with nitrogen. Then, distilled water (5.0 mL), dehydrated ethanol (2.0 mL), and acetophenone (0.12 mL, 1.0 mmol) were sequentially charged, and the mixture was heated to 60°C in an oil bath and stirred at 750 rpm for 2 hours using a stirrer to produce the target (R)-1-phenylethyl alcohol. Conversion rate: 89.9%, selectivity: 100%, optical purity: 96.9%ee. GC retention time (measurement condition 1); acetophenone: 2.99 minutes, (R)-1-phenylethanol: 5.59 minutes, (S)-1-phenylethanol: 6.06 minutes.

Note that when the reaction solution was analyzed by ICP optical emission spectroscopic analysis, 0.1% of ruthenium elution on the activated carbon was observed.

[Example 26] Production of (R)-1-phenylethyl alcohol by asymmetric transfer hydrogenation of acetophenone using a catalyst (catalyst 2) containing a (R,R)-Ts-DENEB complex adsorbed on activated carbon (A2)

The target (R)-1-phenylethyl alcohol was produced in the same manner as in Example 25 except that catalyst 2 (0.43 g, 0.005 mmol, 0.5 mol%) prepared in Example 2 was used as a catalyst. Conversion rate: 99.0%, selectivity: 100%, optical purity: 96.9%ee (according to GC analysis). Here, the conversion rate indicates ([amount of acetophenone charged]-[amount of acetophenone remaining after the reaction])/[amount of acetophenone charged]×100, and the selectivity indicates ([amount of (R)-1-phenylethyl alcohol produced]+[amount of (S)-1-phenylethyl alcohol produced])/([amount of acetophenone charged]-[amount of acetophenone remaining after the reaction])×100. Note that when the reaction solution was analyzed by ICP optical emission spectroscopic analysis, 0.2% of ruthenium elution on the activated carbon was observed.

[Example 27] Production of (R)-1-phenylethyl alcohol by asymmetric transfer hydrogenation of acetophenone using a catalyst (catalyst 3) containing a (R,R)-Ts-DENEB complex adsorbed on activated carbon (B1)

The target (R)-1-phenylethyl alcohol was produced in the same manner as in Example 25 except that catalyst 3 (0.55 g, 0.005 mmol, 0.5 mol%) prepared in Example 3 was used as a catalyst. Conversion rate: 96.2%, selectivity: 100%, optical purity: 96.8%ee (according to GC analysis). Note that when the reaction solution was analyzed by ICP optical emission analysis, 0.2% of ruthenium elution on the activated carbon was observed.

[Example 28] Production of (R)-1-phenylethyl alcohol by asymmetric transfer hydrogenation of acetophenone using a catalyst (catalyst 4) containing a (R,R)-Ts-DENEB complex adsorbed on activated carbon (B2)

The target (R)-1-phenylethyl alcohol was produced in the same manner as in Example 25 except that catalyst 4 (0.77 g, 0.007 mmol, 0.7 mol%) prepared in Example 4 was used as a catalyst. Conversion rate: 97.4%, selectivity: 100%, optical purity: 96.6%ee (according to GC analysis). Note that when the reaction solution was analyzed by ICP optical emission spectroscopic analysis, 0.8% of ruthenium elution on the activated carbon was observed.

[Example 29] Production of (R)-1-phenylethyl alcohol by asymmetric transfer hydrogenation of acetophenone using a catalyst (catalyst 5) containing a (R,R)-Ts-DENEB complex adsorbed on activated carbon (C1)

The target (R)-1-phenylethyl alcohol was produced in the same manner as in Example 25 except that catalyst 5 (0.43 g, 0.005 mmol, 0.5 mol%) prepared in Example 5 was used as a catalyst. (Conversion rate: 0.5%) Note that when the reaction solution was analyzed by ICP optical emission spectroscopic analysis, 0.4% of ruthenium elution on the activated carbon was observed.

[Example 30] Production of (R)-1-phenylethyl alcohol by asymmetric transfer hydrogenation of acetophenone using a catalyst (catalyst 6) containing a (R,R)-Ts-DENEB complex adsorbed on activated carbon (C2)

The target (R)-1-phenylethyl alcohol was produced in the same manner as in Example 25 except that catalyst 6 (0.43 g, 0.005 mmol, 0.5 mol%) prepared in Example 6 was used as a catalyst. Conversion rate: 56.7%, selectivity: 100%, optical purity: 97.1%ee (according to GC analysis). Note that when the reaction solution was analyzed by ICP optical emission spectroscopic analysis, 0.2% of ruthenium elution on the activated carbon was observed.

[Example 31] Production of (R)-1-phenylethyl alcohol by asymmetric transfer hydrogenation of acetophenone using a catalyst (catalyst 7) containing a (R,R)-Ts-DENEB complex adsorbed on activated carbon (M)

The target (R)-1-phenylethyl alcohol was produced in the same manner as in Example 25 except that catalyst

7 (0.43 g, 0.005 mmol, 0.5 mol%) prepared in Example 7 was used as a catalyst. Conversion rate: 95.7%, selectivity: 100%, optical purity: 96.3%ee (according to GC analysis). Note that when the reaction solution was analyzed by ICP optical emission spectroscopic analysis, 0.2% of ruthenium elution on the activated carbon was observed.

[Comparative Example 1] Production of (R)-1-phenylethyl alcohol by asymmetric transfer hydrogenation of acetophenone using (R,R)-Ts-DENEB

The target (R)-1-phenylethyl alcohol was produced in the same manner as in Example 25 except that Ts-DENEB (3.3 mg, 0.005 mmol, 0.5 mol%) was used as a catalyst. Conversion rate: 93.5%, selectivity: 100%, optical purity: 94.4%ee (according to GC analysis).

Table 8 shows the activity test results of activated carbon support catalysts and homogeneous catalysts presented in Examples 25 to 31 and Comparative Example 1.

Table 8: Activity test results of various catalysts and homogeneous catalysts

| | Catalyst No. or Abbreviation Used | Activated Carbon Used | Conversion Rate (%) | Optical Purity (%ee) | Amount of Ruthenium Eluted (%) |
|---|---|---|---|---|---|
| Example 25 | 1 | A1 | 89.9 | 96.9 | 0.1 |
| Example 26 | 2 | A2 | 99.0 | 96.9 | 0.2 |
| Example 27 | 3 | B1 | 96.2 | 96.8 | 0.2 |
| Example 28 | 4 | B2 | 97.4 | 96.6 | 0.8 |
| Example 29 | 5 | C1 | 0.5 | - | 0.4 |
| Example 30 | 6 | C2 | 56.7 | 97.1 | 0.2 |
| Example 31 | 7 | M | 95.7 | 96.3 | 0.2 |
| Comparative Example 1 | Ts-DENEB | - | 93.5 | 94.4 | - |

As described above, it was found that the activated carbon support Ts-DENEB catalysts had a high catalytic activity comparable to that of the homogeneous catalyst, and that ruthenium elution into the reaction solution was hardly observed.

[Example 32] Measurement of change over time in asymmetric transfer hydrogenation of acetophenone using a catalyst (catalyst 3) containing a ruthenium complex adsorbed on activated carbon

(*R,R*)-Ts-DENEB/Activated Carbon (0.16 mol%)
Potassium Formate (20 eq.)

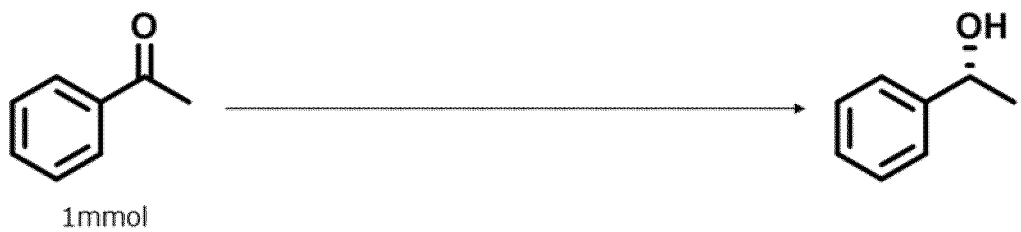


Distilled Water (5 mL)
Dehydrated Ethanol (2 mL)
60°C, 5 Hours

A magnetic stirring bar, catalyst 3 prepared in Example 3 (0.17 g, 0.016 mmol, 0.16 mol%), and potassium

formate (1.68 mmol, 1.68 g, 20 mmol) were charged in an 80 mL Schlenk tube, and the inside of the apparatus was replaced with nitrogen. Then, distilled water (5.0 mL), dehydrated ethanol (2.0 mL), and acetophenone (0.12 mL, 1.0 mmol) were sequentially charged, and the mixture was heated to 60°C in an oil bath and stirred at 750 rpm for 5 hours using a stirrer. 0.1 mL of the suspension being heated was collected with a syringe, and the change over time in the conversion rate was measured. Fig. 1 shows the results.

[Example 33] Hot filtration test in asymmetric transfer hydrogenation of acetophenone using a catalyst (catalyst 3) containing a Ts-DENEB complex adsorbed on activated carbon

Two 80 mL Schlenk tubes were prepared, one of which was charged with a magnetic stirring bar and equipped with a glass filter having a glass fiber filter paper. The other was charged with a magnetic stirring bar, catalyst 3 prepared in Example 3 (0.17 g, 0.016 mmol, 0.16 mol%), and potassium formate (1.68 mmol, 1.68 g, 20 mmol). After that, the inside of the apparatus was replaced with nitrogen. Distilled water (5.0 mL), ethanol (2.0 mL), and acetophenone (0.12 mL, 1.0 mmol) were subsequently charged into the Schlenk tube into which the reagent had been charged. Each Schlenk tube was heated to 60°C in an oil bath and stirred at 750 rpm using a stirrer. One hour after the start of heating and stirring, the suspension was transferred via a cannula to an 80 mL Schlenk tube prepared in advance, and the liquid phase components were filtered into the Schlenk tube under nitrogen. After that, heating and stirring were continued at 60°C. 0.1 mL of the reaction solution being heated was collected with a syringe, and the change over time in the conversion rate was measured. Fig. 2 shows the transition of the conversion rate together with the results of Example 32.

As shown in Fig. 2, when the solid components are removed during the heating reaction, the reaction is completely stopped. That is, it was found that in the present catalytic reaction, not the complex eluted in the liquid phase components worked, but the complex adsorbed on the activated carbon worked.

[Example 34] Production of (R)-1-phenylethyl alcohol by asymmetric transfer hydrogenation of acetophenone using a catalyst (catalyst 8) containing a (R,R)-Ts-DENEB complex adsorbed on activated carbon (A1, granular)

(*R,R*)-Ts-DENEB/Activated Carbon (0.5 mol%)
Potassium Formate (20 eq.)

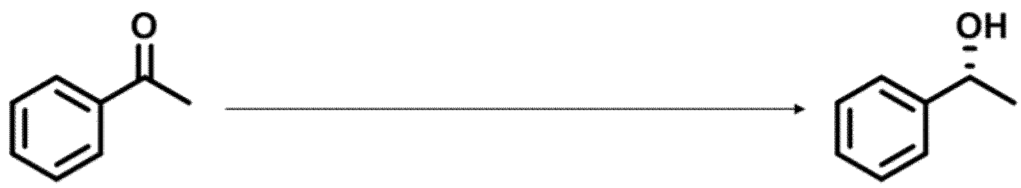

2mmol

Distilled Water (10 mL)
Dehydrated Ethanol (5 mL)
60°C, 2.5 Hours

[Reaction (First Reaction)]

A mechanical stirring paddle, a reflux condenser, a thermometer, and an inlet adapter with 3-way stopcock were attached to a 50 mL four-necked round-bottom flask, and catalyst 8 (0.86 g, 0.01 mmol, 0.5 mol%) prepared in Example 8 and potassium formate (3.36 g, 40 mmol) were charged therein, and the inside of the apparatus was replaced with nitrogen. Then, distilled water (10 mL), ethanol (5.0 mL), and acetophenone (0.23 mL, 2.0 mmol) were sequentially charged, and the mixture was heated to 60°C in an oil bath and stirred at 250 rpm for 2.5 hours using a mechanical stirrer to produce the target (R)-1-phenylethyl alcohol. Conversion rate: 98.3%, selectivity: 100%, optical purity: 97.2%ee (according to GC analysis). In addition, in order to show the change over time, the data for performing the reaction (stirring) for 1 hour was also collected.

[Preparation of Catalyst Cleaning Solution]

Potassium formate in an amount of 67.20 g was changed in a 500 mL round-bottom flask with stopcock, and

the inside was replaced with nitrogen. 200 mL of distilled water and 100 mL of dehydrated ethanol were sequentially charged, and potassium formate was dissolved to prepare a catalyst cleaning solution.

[Catalyst Reuse Operation (Second Reaction)]

After the reaction was completed, stirring was stopped and the supernatant was removed via a syringe. The solid components were washed twice with 5 mL of the prepared catalyst cleaning solution, and compound 8 was recovered. Potassium formate (1.68 g, 40 mmol) was charged in this flask, and the inside of the apparatus was replaced with nitrogen. Then, distilled water (10 mL), dehydrated ethanol (5.0 mL), and acetophenone (0.23 mL, 2.0 mmol) were sequentially charged, and the mixture was heated to 60°C in an oil bath and stirred for 2.5 hours using a mechanical stirrer to produce the target (R)-1-phenylethyl alcohol. Conversion rate: 99.0%, selectivity: 100%, optical purity: 96.6%ee (according to GC analysis). In addition, in order to show the change over time, the data for performing the reaction (stirring) for 1 hour was also collected.

[Catalyst Reuse Operation (Third and Later Reactions)]

The catalyst was reused by the same operation after the second and later reactions.
Table 9 shows the conversion rate, optical purity, and amount of ruthenium eluted for each reaction.

Table 9: Conversion rate, optical purity, and amount of ruthenium eluted for each reaction in Example 34

| | | Reaction Time (Hour) | Conversion Rate (%) | Optical Purity (ee%) | Amount of Ruthenium Eluted (%) |
|---|---|---|---|---|---|
| Experiment 1 | First Reaction | 1 | 57.5 | 97.0 | - |
| | | 2.5 | 98.3 | 97.2 | 0.4 |
| Experiment 2 | Second Reaction | 1 | 83.3 | 96.4 | - |
| | | 2.5 | 99.0 | 96.6 | 0.1 |
| Experiment 3 | Third Reaction | 1 | 78.4 | 96.7 | - |
| | | 2.5 | 98.1 | 96.8 | 0.1 |
| Experiment 4 | Fourth Reaction | 1 | 77.1 | 96.8 | - |
| | | 2.5 | 96.5 | 96.5 | 0.3 |
| Experiment 5 | Fifth Reaction | 1 | 76.9 | 96.4 | - |
| | | 2.5 | 96.4 | 96.4 | <0.1 |

[Example 35] Production of (R)-1-phenylethyl alcohol by asymmetric transfer hydrogenation of acetophenone using a catalyst (catalyst 9) containing a (R,R)-Ts-DENEB complex adsorbed on activated carbon (A2, granular)

The target (R)-1-phenylethyl alcohol was produced by the same operation as in Example 34 except that catalyst 9 (0.86 g, 0.01 mmol, 0.5 mol%) was used as a catalyst and the heating and stirring time was changed to 2 hours. Conversion rate: 99.4%, selectivity: 100%, optical purity: 97.0%ee (according to GC analysis).
The target (R)-1-phenylethyl alcohol was produced by preparing the catalyst cleaning solution and reusing the catalyst under the same conditions as in Example 34 (note that in the present Example, the heating and stirring time was 2 hours). Table 10 shows the conversion rate, optical purity, and amount of ruthenium eluted for each reaction.

Table 10: Conversion rate, optical purity, and amount of ruthenium eluted for each reaction in Example 35

| | | Reaction Time (Hour) | Conversion Rate (%) | Optical Purity (ee%) | Amount of Ruthenium Eluted (%) |
|---|---|---|---|---|---|
| Experiment 1 | First Reaction | 1 | 84.8 | 96.9 | - |
| | | 2 | 99.4 | 97.0 | 0.5 |
| Experiment 2 | Second Reaction | 1 | 90.2 | 97.1 | - |
| | | 2 | 99.0 | 96.7 | <0.1 |
| Experiment 3 | Third Reaction | 1 | 85.5 | 96.2 | - |
| | | 2 | 98.7 | 96.8 | <0.1 |
| Experiment 4 | Fourth Reaction | 1 | 86.3 | 96.6 | - |
| | | 2 | 97.6 | 96.5 | <0.1 |
| Experiment 5 | Fifth Reaction | 1 | 82.9 | 96.8 | - |
| | | 2 | 95.2 | 96.5 | <0.1 |

[Example 36] Production of (R)-1-phenylethyl alcohol by asymmetric transfer hydrogenation of acetophenone using a catalyst (catalyst 10) containing a (R,R)-Ts-DENEB complex adsorbed on activated carbon (B1, granular)

The target (R)-1-phenylethyl alcohol was produced by the same operation as in Example 34 except that catalyst 10 (1.22 g, 0.01 mmol, 0.5 mol%) was used as a catalyst. Conversion rate: 97.5%, selectivity: 100%, optical purity: 97.0%ee (according to GC analysis).

The target (R)-1-phenylethyl alcohol was produced by preparing the catalyst cleaning solution and reusing the catalyst under the same conditions as in Example 34 (note that in the present Example, the heating and stirring time was 2 hours). Table 11 shows the conversion rate, optical purity, and amount of ruthenium eluted for each reaction.

Table 11: Conversion rate, optical purity, and amount of ruthenium eluted for each reaction in Example 36

| | | Reaction Time (Hour) | Conversion Rate (%) | Optical Purity (eel%) | Amount of Ruthenium Eluted (%) |
|---|---|---|---|---|---|
| Experiment 1 | First Reaction | 1 | 65.6 | 97.3 | - |
| | | 2.5 | 97.5 | 97.0 | 1.2 |
| Experiment 2 | Second Reaction | 1 | 79.3 | 96.9 | - |
| | | 2 | 95.9 | 96.7 | 0.7 |
| Experiment 3 | Third Reaction | 1 | 80.0 | 96.2 | - |
| | | 2 | 95.6 | 96.3 | 0.4 |
| Experiment 4 | Fourth Reaction | 1 | 72.9 | 96.5 | - |
| | | 2 | 89.0 | 96.1 | 0.2 |
| Experiment 5 | Fifth Reaction | 1 | 77.5 | 96.4 | - |
| | | 2 | 90.3 | 96.4 | 0.2 |

[Example 37] Production of (R)-1-phenylethyl alcohol by asymmetric transfer hydrogenation of acetophenone using a catalyst (catalyst11) containing a (R,R)-Ts-DENEB complex adsorbed on activated carbon (B2, granular)

The target (R)-1-phenylethyl alcohol was produced by the same operation as in Example 34 except that catalyst 11 (1.22 g, 0.01 mmol, 0.5 mol%) was used as a catalyst and the heating and stirring time was changed to 2 hours. Conversion rate: 96.9%, selectivity: 100%, optical purity: 96.7%ee (according to GC analysis).

The target (R)-1-phenylethyl alcohol was produced by preparing the catalyst cleaning solution and reusing the catalyst under the same conditions as in Example 34 (note that in the present Example, the heating and stirring time was 2 hours). Table 12 shows the conversion rate, optical purity, and amount of ruthenium eluted for each reaction.

Table 12: Conversion rate, optical purity, and amount of ruthenium eluted for each reaction in Example 37

| | | Reaction Time (Hour) | Conversion Rate (%) | Optical Purity (ee%) | Amount of Ruthenium Eluted (%) |
|---|---|---|---|---|---|
| Experiment 1 | First Reaction | 1 | 76.1 | 96.9 | - |
| | | 2 | 96.9 | 96.7 | <0.1 |
| Experiment 2 | Second Reaction | 1 | 88.3 | 96.6 | - |
| | | 2 | 96.6 | 96.4 | <0.1 |
| Experiment 3 | Third Reaction | 1 | 82.9 | 96.5 | - |
| | | 2 | 95.6 | 96.2 | <0.1 |
| Experiment 4 | Fourth Reaction | 1 | 77.6 | 96.2 | - |
| | | 2 | 93.0 | 96.3 | <0.1 |
| Experiment 5 | Fifth Reaction | 1 | 75.1 | 96.0 | - |
| | | 2 | 90.0 | 95.7 | <0.1 |
| Experiment 6 | Sixth Reaction | 1 | 67.4 | 96.3 | - |
| | | 2 | 87.0 | 96.3 | <0.1 |

[Example 38] Production of (R)-1-phenylethyl alcohol by asymmetric transfer hydrogenation of acetophenone using a catalyst (catalyst 15) containing a (R,R)-Ts-DENEB complex adsorbed on activated carbon (A2, granular)

The target (R)-1-phenylethyl alcohol was produced by the same operation as in Example 34 except that catalyst 15 (171.1 mg, 0.01 mmol, 0.5 mol%) was used as a catalyst and the heating and stirring time was changed to 2 hours. Conversion rate: 99.7%, selectivity: 100%, optical purity: 97.1%ee (according to GC analysis).

The target (R)-1-phenylethyl alcohol was produced by preparing the catalyst cleaning solution and reusing the catalyst under the same conditions as in Example 34 (note that in the present Example, the heating and stirring time was 2 hours). Table 13 shows the conversion rate, optical purity, and amount of ruthenium eluted for each reaction.

Table 13: Conversion rate, optical purity, and amount of ruthenium eluted for each reaction in Example 38

| | | Reacti on Time (Hour) | Conversion Rate (%) | Optical Purity (ee%) | Amount of Ruthenium Eluted (%) |
|---|---|---|---|---|---|
| Experiment 1 | First Reaction | 1 | 75.7 | 97.2 | - |
| | | 2 | 99.7 | 97.1 | <0.1 |

(continued)

| | | Reacti on Time (Hour) | Conversion Rate (%) | Optical Purity (ee%) | Amount of Ruthenium Eluted (%) |
|---|---|---|---|---|---|
| Experiment 2 | Second Reaction | 1 | 78.6 | 97.1 | - |
| | | 2 | 99.9 | 96.9 | <0.1 |
| Experiment 3 | Third Reaction | 1 | 78.1 | 97.0 | - |
| | | 2 | 99.5 | 96.9 | <0.1 |
| Experiment 4 | Fourth Reaction | 1 | 78.5 | 96.9 | - |
| | | 2 | 99.5 | 96.7 | <0.1 |
| Experiment 5 | Fifth Reaction | 1 | 72.5 | 96.8 | - |
| | | 2 | 98.8 | 97.0 | <0.1 |
| Experiment 6 | Sixth Reaction | 1 | 71.0 | 97.0 | - |
| | | 2 | 98.5 | 95.9 | <0.1 |
| Experiment 7 | Seventh Reaction | 1 | 64.5 | 96.9 | - |
| | | 2 | 97.6 | 96.4 | <0.1 |

[Example 39] Production of (R)-1-phenylethyl alcohol by asymmetric transfer hydrogenation of acetophenone using a catalyst (catalyst 16) containing a (R,R)-Ts-DENEB complex adsorbed on activated carbon (A2, granular)

The target (R)-1-phenylethyl alcohol was produced by the same operation as in Example 34 except that catalyst 16 (97.0 mg, 0.01 mmol, 0.5 mol%) was used as a catalyst and the heating and stirring time was changed to 3 hours. Conversion rate: 99.2%, selectivity: 100%, optical purity: 96.9%ee (according to GC analysis).

The target (R)-1-phenylethyl alcohol was produced by preparing the catalyst cleaning solution and reusing the catalyst under the same conditions as in Example 34 (note that in the present Example, the heating and stirring time was 3 hours). Table 14 shows the conversion rate, optical purity, and amount of ruthenium eluted for each reaction.

Table 14: Conversion rate, optical purity, and amount of ruthenium eluted for each reaction in Example 39

| | | Reaction Time (Hour) | Conversion Rate (%) | Optical Purity (ee%) | Amount of Ruthenium Eluted (%) |
|---|---|---|---|---|---|
| Experiment 1 | First Reaction | 1 | 39.8 | 96.2 | - |
| | | 3 | 99.2 | 96.9 | <0.1 |
| Experiment 2 | Second Reaction | 1 | 53.1 | 97.2 | - |
| | | 3 | 99.8 | 97.1 | <0.1 |
| Experiment 3 | Third Reaction | 1 | 53.8 | 97.2 | - |
| | | 3 | 99.5 | 97.0 | <0.1 |
| Experiment 4 | Fourth Reaction | 1 | 56.1 | 96.3 | - |
| | | 2.5 | 97.9 | 97.1 | <0.1 |

(continued)

| | | Reaction Time (Hour) | Conversion Rate (%) | Optical Purity (ee%) | Amount of Ruthenium Eluted (%) |
|---|---|---|---|---|---|
| Experiment 5 | Fifth Reaction | 1 | 50.6 | 96.7 | - |
| | | 3 | 99.4 | 96.8 | <0.1 |
| Experiment 6 | Sixth Reaction | 1 | 51.8 | 96.8 | - |
| | | 3 | 99.5 | 97.0 | <0.1 |
| Experiment 7 | Seventh Reaction | 1 | 47.4 | 96.9 | - |
| | | 3 | 99.6 | 96.9 | <0.1 |

[Example 40] Production of (R)-1-phenylethyl alcohol by asymmetric transfer hydrogenation of acetophenone using a catalyst (catalyst 17) containing a (R,R)-Ts-DENEB complex adsorbed on activated carbon (C2, granular)

The target (R)-1-phenylethyl alcohol was produced by the same operation as in Example 34 except that catalyst 17 (90.9 mg, 0.01 mmol, 0.5 mol%) was used as a catalyst. Conversion rate: 99.7%, selectivity: 100%, optical purity: 97.7%ee (according to GC analysis).

The target (R)-1-phenylethyl alcohol was produced by preparing the catalyst cleaning solution and reusing the catalyst under the same conditions as in Example 34 (note that in the present Example, the heating and stirring time was 3 hours). Table 15 shows the conversion rate, optical purity, and amount of ruthenium eluted for each reaction.

Table 15: Conversion rate, optical purity, and amount of ruthenium eluted for each reaction in Example 40

| | | Reaction Time (Hour) | Conversion Rate (%) | Optical Purity (ee%) | Amount of Ruthenium Eluted (%) |
|---|---|---|---|---|---|
| Experiment 1 | First Reaction | 1 | 68.2 | 97.6 | - |
| | | 2.5 | 99.9 | 97.7 | <0.1 |
| Experiment 2 | Second Reaction | 1 | 62.3 | 97.5 | - |
| | | 2.5 | 99.7 | 97.6 | <0.1 |
| Experiment 3 | Third Reaction | 1 | 57.9 | 97.5 | - |
| | | 3 | 99.0 | 97.6 | 0.1 |
| Experiment 4 | Fourth Reaction | 1 | 40.0 | 97.2 | - |
| | | 3 | 97.1 | 97.6 | <0.1 |
| Experiment 5 | Fifth Reaction | 1 | 42.7 | 97.0 | - |
| | | 3 | 96.0 | 97.4 | <0.1 |

As described above, it was found that the activated carbon support ruthenium complex of the present invention maintained high catalytic activity even for reuse after catalyst recovery. In addition, it was found that the amount of ruthenium eluted in each reaction solution was very small, and the ruthenium complex was firmly supported on the activated carbon.

[Example 41] Production of (R)-1-phenylethyl alcohol by asymmetric transfer hydrogenation of acetophenone using a catalyst (catalyst 20) containing a (R,R)-Ts-DENEB complex adsorbed on activated carbon (C2, granular)

The target (R)-1-phenylethyl alcohol was produced by the same operation as in Example 34 except that catalyst 20 (36.3 mg, 0.01 mmol, 0.5 mol%) was used as a catalyst and the heating and stirring time was changed to 2 hours. Conversion rate: 100.0%, selectivity: 100%, optical purity: 97.5%ee (according to GC analysis).

The target (R)-1-phenylethyl alcohol was produced by preparing the catalyst cleaning solution and reusing the catalyst under the same conditions as in Example 34 (note that in the present Example, the heating and stirring time was 3 hours). Table 16 shows the conversion rate, optical purity, and amount of ruthenium eluted for each reaction.

Table 16: Conversion rate, optical purity, and amount of ruthenium eluted for each reaction in Example 41

| | | Reaction Time (Hour) | Conversion Rate (%) | Optical Purity (ee%) | Amount of Ruthenium Eluted (%) |
|---|---|---|---|---|---|
| Experiment 1 | First Reaction | 1 | 77.5 | 97.2 | - |
| | | 2 | 100.0 | 97.5 | 5.0 |
| Experiment 2 | Second Reaction | 1 | 54.3 | 97.3 | - |
| | | 2 | 92.9 | 97.5 | 2.2 |

As is apparent from the comparison between Examples 40 and 41, when the amount of the (R,R)-Ts-DENEB complex adsorbed on activated carbon is increased, the amount of ruthenium eluted after the reaction tends to increase. Therefore, from the viewpoint of practicality, it is desirable that the total mass of the (R,R)-Ts-DENEB complex to be adsorbed is 25% by mass or less based on the total mass of the activated carbon.

[Example 42] Production of (R)-1-phenylethyl alcohol by asymmetric transfer hydrogenation of acetophenone using a catalyst (catalyst 21) containing (R,R)-Ms-DENEB complex (manufactured by Takasago International Corporation) adsorbed on activated carbon (C2, granular)

The target (R)-1-phenylethyl alcohol was produced by the same operation as in Example 34 except that catalyst 21 (100.7 mg, 0.01 mmol, 0.5 mol%) was used as a catalyst. Conversion rate: 100%, selectivity: 100%, optical purity: 96.6%ee (according to GC analysis).

The target (R)-1-phenylethyl alcohol was produced by preparing the catalyst cleaning solution and reusing the catalyst under the same conditions as in Example 34 (note that in the present Example, the heating and stirring time was 2 hours). Table 17 shows the conversion rate, optical purity, and amount of ruthenium eluted for each reaction.

Table 17: Conversion rate, optical purity, and amount of ruthenium eluted for each reaction in Example 42

| | | Reaction Time (Hour) | Conversion Rate (%) | Optical Purity (ee%) | Amount of Ruthenium Eluted (%) |
|---|---|---|---|---|---|
| Experiment 1 | First Reaction | 1 | 69.6 | 96.8 | - |
| | | 2.5 | 100 | 96.6 | <0.1 |
| Experiment 2 | Second Reaction | 1 | 57.5 | 96.3 | - |
| | | 2 | 91.2 | 96.3 | - |

As shown in Table 17, it was found that Ms-DENEB, which has a tether structure similar to Ts-DENEB, also had high reusability when supported on activated carbon without elution of ruthenium into the reaction solution.

[Example 43] Production of (R)-1-phenylethyl alcohol by asymmetric transfer hydrogenation of acetophenone using a catalyst (catalyst 23) containing a RuCl((R,R)-Ts-DPEN) (p-cymene) complex adsorbed on activated carbon (C2, granular)

The target (R)-1-phenylethyl alcohol was produced by the same operation as in Example 34 except that catalyst 23 (120.0 mg, 0.01 mmol, 0.5 mol%) was used as a catalyst. Conversion rate: 94.3%, selectivity: 100%, optical purity: 94.8%ee (according to GC analysis).

The target (R)-1-phenylethyl alcohol was produced by preparing the catalyst cleaning solution and reusing the catalyst under the same conditions as in Example 34 (note that in the present Example, the heating and stirring time was 2 hours). Table 18 shows the conversion rate, optical purity, and amount of ruthenium eluted for each reaction.

Table 18: Conversion rate, optical purity, and amount of ruthenium eluted for each reaction in Example 43

| | | Reaction Time (Hour) | Conversion Rate (%) | Optical Purity (ee%) | Amount of Ruthenium Eluted (%) |
|---|---|---|---|---|---|
| Experiment 1 | First Reaction | 1 | 55.8 | 94.6 | - |
| | | 2.5 | 94.3 | 94.8 | <0.1 |
| Experiment 2 | Second Reaction | 1 | 28.1 | 93.8 | - |
| | | 2 | 51.9 | 94.3 | - |

As shown in Table 18, it was found that RuCl((R,R)-Ts-DPEN) (p-cymene), which is a general-purpose complex effective for asymmetric transfer hydrogenation, also had moderate reusability when supported on activated carbon without elution of ruthenium into the reaction solution.

Next, the effects on the reaction conversion rate and optical purity in the production of (R)-1-phenylethyl alcohol by the asymmetric transfer hydrogenation of acetophenone caused by the catalyst of the present invention were compared with Comparative Examples 2 and 3 below. Table 19 shows the results.

[Comparative Example 2] Production of (R)-1-phenylethyl alcohol by asymmetric transfer hydrogenation of acetophenone using (R,R)-Ts-DENEB complex

A magnetic stirring bar, Ts-DENEB (6.5 mg, 0.01 mmol, 0.5 mol%), and potassium formate (3.36 g, 40 mmol) were charged in an 80 mL Schlenk tube, and the inside of the apparatus was replaced with nitrogen. Then, distilled water (10 mL), ethanol (5.0 mL), and acetophenone (0.23 mL, 2.0 mmol) were sequentially charged, and the mixture was heated to 60°C in an oil bath and stirred at 750 rpm for 2.5 hours using a stirrer to produce the target (R)-1-phenylethyl alcohol. Table 19 shows the reaction conversion rate and the optical purity calculated from the GC analysis results.

[Comparative Example 3] Production of (R)-1-phenylethyl alcohol by asymmetric transfer hydrogenation of acetophenone using (R,R)-Ts-DENEB complex in the presence of formic acid-triethylamine

A magnetic stirring bar and (R,R)-Ts-DENEB (6.5 mg, 0.01 mmol, 0.5 mol%) was charged into an 80 mL Schlenk tube, and the inside of the apparatus was replaced with nitrogen. Then, acetophenone (0.23 mL, 2.0 mmol) and a formic acid-triethylamine mixed solution (5:2, 2.0 mL, 20 mmol) were sequentially charged, and the mixture was heated to 60°C in an oil bath and stirred at 750 rpm for 1 hour using a stirrer to produce the target (R)-1-phenylethyl alcohol. Table 19 shows the reaction conversion rate and the optical purity calculated from the GC analysis results.

Table 19: Reaction conversion rate and the optical purity calculated from the GC analysis results

| | Catalyst | Solvent | Reductant | Reaction Time (Hour) | Conversion Rate (%) | Optical Purity (%ee) |
|---|---|---|---|---|---|---|
| Example 41 | Ts-DENEB/A ctivated Carbon C2 (Granular) | Water-Ethanol | Potassium Formate | 2.5 | 99.9 | 97.7 |

(continued)

| | Catalyst | Solvent | Reductant | Reaction Time (Hour) | Conversion Rate (%) | Optical Purity (%ee) |
|---|---|---|---|---|---|---|
| Example 39 | Ts-DENEB/A ctivated Carbon A2 (Granular) | Water-Ethanol | Potassium Formate | 3 | 99.2 | 96.9 |
| Comparative Example 2 | Ts-DENEB | Water-Ethanol | Potassium Formate | 2.5 | 97.8 | 93.0 |
| Comparative Example 3 | Ts-DENEB | N/A | Formic Acid-Triethylamine | 1 | 96.6 | 96.1 |

The effects on the reaction conversion rate and optical purity in the production of (R)-1-(4'-chlorophenyl)ethanol by the asymmetric transfer hydrogenation of 4'-chloroacetophenone caused by the catalyst of the present invention were examined. Table 20 shows the results.

[Example 44] Production of (R)-1-(4'-chlorophenyl)ethanol by asymmetric transfer hydrogenation of 4'-chloroacetophenone using a catalyst (catalyst 17) containing a (R,R)-Ts-DENEB complex adsorbed on activated carbon (C2, granular)

The target (R)-1-(4'-chlorophenyl)ethanol was produced by the same operation as in Example 40 except that 4'-chloroacetophenone (0.31 g, 2.0 mmol) was used as a raw material, and the heating and stirring time was changed to 5 hours. Table 20 shows the reaction conversion rate and the optical purity calculated from the GC analysis results.

GC retention time (measurement condition 2); 4'-chloroacetophenone: 7.36 minutes, (R)-1-(4'-chlorophenyl)ethanol: 19.58 minutes, (S)-1-(4'-chlorophenyl)ethanol: 22.76 minutes.

[Example 45] Production of (R)-1-(4'-chlorophenyl)ethanol by asymmetric transfer hydrogenation of 4'-chloroacetophenone using a catalyst (catalyst 16) containing a (R,R)-Ts-DENEB complex adsorbed on activated carbon (A2, granular)

The target (R)-1-(4'-chlorophenyl)ethanol was produced by the same operation as in Example 39 except that 4'-chloroacetophenone (0.31 g, 2.0 mmol) was used as a raw material, and the heating and stirring time was changed to 5 hours. Table 20 shows the reaction conversion rate and the optical purity calculated from the GC analysis results.

[Comparative Example 4] Production of (R)-1-(4'-chlorophenyl)ethanol by asymmetric transfer hydrogenation of 4'-chloroacetophenone using a (R,R)-Ts-DENEB complex

The target (R)-1-(4'-chlorophenyl)ethanol was produced by the same operation as in Comparative Example 2 except that 4'-chloroacetophenone (0.31 g, 2.0 mmol) was used as a raw material, and the heating and stirring time was changed to 5 hours. Table 20 shows the reaction conversion rate and the optical purity calculated from the GC analysis results.

[Comparative Example 5] Production of (R)-1-(4'-chlorophenyl)ethanol by asymmetric transfer hydrogenation of 4'-chloroacetophenone using a (R,R)-Ts-DENEB complex in the presence of formic acid-triethylamine

The target (R)-1-(4'-chlorophenyl)ethanol was produced by the same operation as in Comparative Example 3 except that 4'-chloroacetophenone (0.31 g, 2.0 mmol) was used as a raw material, and the heating and stirring time was changed to 3 hours. Table 20 shows the reaction conversion rate and the optical purity calculated from the GC analysis results.

Table 20: Reaction conversion rate and the optical purity calculated from the GC analysis results

| | Catalyst | Solvent | Reductant | Reaction Time (Hour) | Conversion Rate (%) | Optical Purity (%ee) |
|---|---|---|---|---|---|---|
| Example 44 | Ts-DENEB/Activated Carbon C2 (Granular) | Water-Ethanol | Potassium Formate | 5 | 99.3 | 93.5 |
| Example 45 | Ts-DENEB/Activated Carbon A2 (Granular) | Water-Ethanol | Potassium Formate | 5 | 99.1 | 90.4 |
| Comparative Example 4 | Ts-DENEB | Water-Ethanol | Potassium Formate | 5 | 99.8 | 91.3 |
| Comparative Example 5 | Ts-DENEB | N/A | Formic Acid-Triethylamine | 3 | 99.8 | 91.8 |

The effects on the reaction conversion rate and optical purity in the production of (R)-1-(2'-chlorophenyl)ethanol by the asymmetric transfer hydrogenation of 2'-chloroacetophenone caused by the catalyst of the present invention were examined. Table 21 shows the results.

[Example 46] Production of (R)-1-(2'-chlorophenyl)ethanol by asymmetric transfer hydrogenation of 2'-chloroacetophenone using a catalyst (catalyst 17) containing a (R,R)-Ts-DENEB complex adsorbed on activated carbon (C2, granular)

The target (R)-1-(2'-chlorophenyl)ethanol was produced by the same operation as in Example 40 except that 2'-chloroacetophenone (0.31 g, 2.0 mmol) was used as a raw material, and the heating and stirring time was changed to 5 hours. Table 21 shows the reaction conversion rate and the optical purity calculated from the GC analysis results.

GC retention time (measurement condition 2); 2'-chloroacetophenone: 5.08 minutes, (R)-1-(2'-chlorophenyl)ethanol: 17.97 minutes, (S)-1-(2'-chlorophenyl)ethanol: 24.83 minutes.

[Example 47] Production of (R)-1-(2'-chlorophenyl)ethanol by asymmetric transfer hydrogenation of 2'-chloroacetophenone using a catalyst (catalyst 16) containing a (A2, granular) (R,R)-Ts-DENEB complex adsorbed on activated carbon

The target (R)-1-(2'-chlorophenyl)ethanol was produced by the same operation as in Example 39 except that 2'-chloroacetophenone (0.31 g, 2.0 mmol) was used as a raw material, and the heating and stirring time was changed to 5 hours. Table 21 shows the reaction conversion rate and the optical purity calculated from the GC analysis results.

[Comparative Example 6] Production of (R)-1-(2'-chlorophenyl)ethanol by asymmetric transfer hydrogenation of 2'-chloroacetophenone using a (R,R)-Ts-DENEB complex

The target (R)-1-(2'-chlorophenyl)ethanol was produced by the same operation as in Comparative Example 2 except that 2'-chloroacetophenone (0.31 g, 2.0 mmol) was used as a raw material, and the heating and stirring time was changed to 5 hours. Table 21 shows the reaction conversion rate and the optical purity calculated from the GC analysis results.

[Comparative Example 7] Production of (R)-1-(2'-chlorophenyl)ethanol by asymmetric transfer hydrogenation of 2'-chloroacetophenone using a (R,R)-Ts-DENEB complex in the presence of formic acid-triethylamine

The target (R)-1-(2'-chlorophenyl)ethanol was produced by the same operation as in Comparative Example 3 except that 2'-chloroacetophenone (0.31 g, 2.0 mmol) was used as a raw material, and the heating and stirring time was changed to 3 hours. Table 21 shows the reaction conversion rate and the optical purity calculated from the GC analysis results.

Table 21: Reaction conversion rate and the optical purity calculated from the GC analysis results

| | | | | | | |
|---|---|---|---|---|---|---|
| | | | | | | |

(continued)

| | Catalyst | Solvent | Reductant | Reactio n Time (Hour) | Conversion Rate (%) | Optical Purity (%ee) |
|---|---|---|---|---|---|---|
| Example 46 | Ts-DENEB/Act ivated Carbon C2 (Granular) | Water-Ethanol | Potassium Formate | 5 | 99.7 | 90.3 |
| Example 47 | Ts-DENEB/Act ivated Carbon A2 (Granular) | Water-Ethanol | Potassium Formate | 5 | 99.4 | 85.0 |
| Comparative Example 6 | Ts-DENEB | Water-Ethanol | Potassium Formate | 5 | 98.3 | 79.1 |
| Comparative Example 7 | Ts-DENEB | N/A | Formic Acid-Triethylami ne | 3 | 97.6 | 86.3 |

The effects on the reaction conversion rate and optical purity in the production of (R)-1-(4'-methoxyphenyl)ethanol by the asymmetric transfer hydrogenation of 4'-methoxyacetophenone caused by the catalyst of the present invention were estimated. Table 22 shows the results.

[Example 48] Production of (R)-1-(4'-methoxyphenyl)ethanol by asymmetric transfer hydrogenation of 4'-methoxyacetophenone using a (R,R)-Ts-DENEB complex (catalyst 17) adsorbed on activated carbon (C2, granular)

The target (R)-1-(4'-methoxyphenyl)ethanol was produced by the same operation as in Example 40 except that 4'-methoxyacetophenone (0.30 g, 2.0 mmol) was used as a raw material, and the heating and stirring time was changed to 5 hours. Table 22 shows the reaction conversion rate and the optical purity calculated from the GC analysis results.

GC retention time (measurement condition 2); 4'-methoxyacetophenone: 12.65 minutes, (R)-1-(4'-methoxyphenyl)ethanol: 19.09 minutes, (S)-1-(4'-methoxyphenyl)ethanol: 20.76 minutes.

[Example 49] Production of (R)-1-(4'-methoxyphenyl)ethanol by asymmetric of 4'-methoxyacetophenone using a catalyst (catalyst 16) containing a (R,R)-Ts-DENEB complex adsorbed on activated carbon (A2, granular)

The target (R)-1-(4'-methoxyphenyl)ethanol was produced by the same operation as in Example 39 except that 4'-methoxyacetophenone (0.30 g, 2.0 mmol) was used as a raw material, and the heating and stirring time was changed to 5 hours. Table 22 shows the reaction conversion rate and the optical purity calculated from the GC analysis results.

[Comparative Example 8] Production of (R)-1-(4'-methoxyphenyl)ethanol by asymmetric transfer hydrogenation of 4'-methoxyacetophenone using a (R,R)-Ts-DENEB complex

The target (R)-1-(4'-methoxyphenyl)ethanol was produced by the same operation as in Comparative Example 2 except that 4'-methoxyacetophenone (0.30 g, 2.0 mmol) was used as a raw material, and the heating and stirring time was changed to 5 hours. Table 22 shows the reaction conversion rate and the optical purity calculated from the GC analysis results.

[Comparative Example 9] Production of (R)-1-(4'-methoxyphenyl)ethanol by asymmetric transfer hydrogenation of 4'-methoxyacetophenone using a (R,R)-Ts-DENEB complex in the presence of formic acid-triethylamine

The target (R)-1-(4'-methoxyphenyl)ethanol was produced by the same operation as in Comparative Example 3 except that 4'-methoxyacetophenone (0.30 g, 2.0 mmol) was used as a raw material, and the heating and stirring time was changed to 3 hours. Table 22 shows the reaction conversion rate and the optical purity calculated from the GC analysis results.

Table 22: Reaction conversion rate and the optical purity calculated from the GC analysis results

| | Catalyst | Solvent | Reductant | Reaction Time (Hour) | Conversion Rate (%) | Optical Purity (%ee) |
|---|---|---|---|---|---|---|
| Example 48 | Ts-DENEB/Act | Water-Ethanol | Potassium Formate | 5 | 99.0 | 95.1 |
| | ivated Carbon C2 (Granular) | | | | | |
| Example 49 | Ts-DENEB/Act ivated Carbon A2 (Granular) | Water-Ethanol | Potassium Formate | 5 | 96.4 | 89.8 |
| Comparative Example 8 | Ts-DENEB | Water-Ethanol | Potassium Formate | 5 | 85.5 | 91.8 |
| Comparative Example 9 | Ts-DENEB | N/A | Formic Acid-Triethyla mine | 3 | 94.1 | 96.0 |

The effects on the reaction conversion rate and optical purity in the production of (R)-1-(2'-methoxyphenyl)ethanol by the asymmetric transfer hydrogenation of 2'-methoxyacetophenone caused by the catalyst of the present invention were estimated. Table 23 shows the results.

[Example 50] Production of (R)-1-(2'-methoxyphenyl)ethanol by asymmetric transfer hydrogenation of 2'-methoxyacetophenone using a catalyst (catalyst 17) containing a (R,R)-Ts-DENEB complex adsorbed on activated carbon (C2, granular)

The target (R)-1-(2'-methoxyphenyl)ethanol was produced by the same operation as in Example 40 except that 2'-methoxyacetophenone (0.30 g, 2.0 mmol) was used as a raw material, and the heating and stirring time was changed to 5 hours. Table 23 shows the reaction conversion rate and the optical purity calculated from the GC analysis results.

GC retention time (measurement condition 2); 2'-methoxyacetophenone: 7.90 minutes, (R)-1-(2'-methoxyphenyl)ethanol: 18.29 minutes, (S)-1-(2'-methoxyphenyl)ethanol: 16.78 minutes.

[Example 51] Production of (R)-1-(2'-methoxyphenyl)ethanol by asymmetric transfer hydrogenation of 2'-methoxyacetophenone using a catalyst (catalyst 16) containing a (R,R)-Ts-DENEB complex adsorbed on activated carbon (A2, granular)

The target (R)-1-(2'-methoxyphenyl)ethanol was produced by the same operation as in Example 39 except that 2'-methoxyacetophenone (0.30 g, 2.0 mmol) was used as a raw material, and the heating and stirring time was changed to 5 hours. Table 23 shows the reaction conversion rate and the optical purity calculated from the GC analysis results.

[Comparative Example 10] Production of (R)-1-(2'-methoxyphenyl)ethanol by asymmetric transfer hydrogenation of 2'-methoxyacetophenone using a (R,R)-Ts-DENEB complex

The target (R)-1-(2'-methoxyphenyl)ethanol was produced by the same operation as in Comparative Example 2 except that 2'-methoxyacetophenone (0.30 g, 2.0 mmol) was used as a raw material, and the heating and stirring time was changed to 5 hours. Table 23 shows the reaction conversion rate and the optical purity calculated from the GC analysis results.

[Comparative Example 11] Production of (R)-1-(2'-methoxyphenyl)ethanol by asymmetric transfer hydrogenation of 2'-methoxyacetophenone using a (R,R)-Ts-DENEB complex in the presence of formic acid-triethylamine

The target (R)-1-(2'-methoxyphenyl)ethanol was produced by the same operation as in Comparative Example 3 except that 2'-methoxyacetophenone (0.30 g, 2.0 mmol) was used as a raw material, and the heating and stirring time was changed to 5 hours. Table 23 shows the reaction conversion rate and the optical purity calculated from the GC analysis results.

Table 23: Reaction conversion rate and the optical purity calculated from the GC analysis results

| | Catalyst | Solvent | Reductant | Reaction Time (Hour) | Conversion Rate (%) | Optical Purity (%ee) |
|---|---|---|---|---|---|---|
| Example 50 | Ts-DENEB/Act ivated Carbon C2 (Granular) | Water-Ethanol | Potassium Formate | 5 | 99.8 | 96.6 |
| Example 51 | Ts-DENEB/Act ivated Carbon A2 (Granular) | Water-Ethanol | Potassium Formate | 5 | 93.0 | 95.1 |
| Comparative Example 10 | Ts-DENEB | Water-Ethanol | Potassium Formate | 5 | 80.0 | 87.3 |
| Comparative Example 11 | Ts-DENEB | N/A | Formic Acid-Triethyla mine | 3 | 94.6 | 94.0 |

The effects on the reaction conversion rate and optical purity in the production of (R)-1-(4'-trifluoromethylphenyl)ethanol by the asymmetric transfer hydrogenation of 4'-trifluoromethylacetophenone caused by the catalyst of the present invention were examined. Table 24 shows the results.

[Example 52] Production of (R)-1-(4'-trifluoromethylphenyl)ethanol by asymmetric transfer hydrogenation of 4'-trifluoromethylacetophenone using a catalyst (catalyst 17) containing a (R,R)-Ts-DENEB complex adsorbed on activated carbon (C2, granular)

The target (R)-1-(4'-trifluoromethylphenyl)ethanol was produced by the same operation as in Example 40 except that 4'-trifluoromethylacetophenone (0.38 g, 2.0 mmol) was used as a raw material, and the heating and stirring time was changed to 5 hours. Table 24 shows the reaction conversion rate and the optical purity calculated from the GC analysis results.

GC retention time (measurement condition 1); 4'-trifluoromethylacetophenone: 3.09 minutes, (R)-1-(4'-trifluoromethylphenyl)ethanol: 10.18 minutes, (S)-1-(4'-trifluoromethylphenyl)ethanol: 13.03 minutes.

[Example 53] Production of (R)-1-(4'-trifluoromethylphenyl)ethanol by asymmetric transfer hydrogenation of 4'-trifluoromethylacetophenone using a catalyst (catalyst 16) containing a (R,R)-Ts-DENEB complex adsorbed on activated carbon (A2, granular)

The target (R)-1-(4'-trifluoromethylphenyl)ethanol was produced by the same operation as in Example 40 except that 4'-trifluoromethylacetophenone (0.38 g, 2.0 mmol) was used as a raw material, and the heating and stirring time was changed to 5 hours. Table 24 shows the reaction conversion rate and the optical purity calculated from the GC analysis results.

[Comparative Example 12] Production of (R)-1-(4'-trifluoromethylphenyl)ethanol by asymmetric transfer hydrogenation of 4'-trifluoromethylacetophenone using a (R,R)-Ts-DENEB complex

The target (R)-1-(4'-trifluoromethylphenyl)ethanol was produced by the same operation as in Comparative Example 2 except that 4'-trifluoromethylacetophenone (0.38 g, 2.0 mmol) was used as a raw material, and the heating and stirring time was changed to 5 hours. Table 24 shows the reaction conversion rate and the optical purity calculated from the GC analysis results.

[Comparative Example 13] Production of (R)-1-(4'-trifluoromethylphenyl)ethanol by asymmetric transfer hydrogenation of 4'-trifluoromethylacetophenone using a (R,R)-Ts-DENEB complex in the presence of formic acid-triethylamine

The target (R)-1-(4'-trifluoromethylphenyl)ethanol was produced by the same operation as in Comparative Example 3 except that 4'-trifluoromethylacetophenone (0.38 g, 2.0 mmol) was used as a raw material, and the heating and stirring time was changed to 3 hours. Table 24 shows the reaction conversion rate and the optical purity calculated from the GC analysis results.

Table 24: Reaction conversion rate and the optical purity calculated from the GC analysis results

| | Catalyst | Solvent | Reductant | Reaction Time (Hour) | Conversion Rate (%) | Optical Purity (%ee) |
|---|---|---|---|---|---|---|
| Example 52 | Ts-DENEB/Act ivated Carbon C2 (Granular) | Water-Ethanol | Potassium Formate | 5 | 100 | 88.2 |
| Example 53 | Ts-DENEB/Act ivated Carbon A2 (Granular) | Water-Ethanol | Potassium Formate | 5 | 85 | 79.5 |
| Comparative Example 12 | Ts-DENEB | Water-Ethanol | Potassium Formate | 5 | 100 | 91.7 |
| Comparative Example 13 | Ts-DENEB | N/A | Formic Acid-Triethylamin e | 3 | 99.6 | 91.7 |

The effects on the reaction conversion rate and optical purity in the production of (R)-1-(4'-cyanophenyl)ethanol by the asymmetric transfer hydrogenation of 4'-cyanoacetophenone caused by the catalyst of the present invention were examined. Table 25 shows the results.

[Example 54] Production of (R)-1-(4'-cyanophenyl)ethanol by asymmetric transfer hydrogenation of 4'-cyanoacetophenone using a catalyst (catalyst 17) containing a (R,R)-Ts-DENEB complex adsorbed on activated carbon (C2, granular)

The target (R)-1-(4'-cyanophenyl)ethanol was produced by the same operation as in Example 40 except that 4'-cyanoacetophenone (0.29 g, 2.0 mmol) was used as a raw material, and the heating and stirring time was changed to 5 hours. Table 25 shows the reaction conversion rate and the optical purity calculated from the GC analysis results.

GC retention time (measurement condition 3); 4'-cyanoacetophenone: 6.05 minutes, (R)-1-(4'-cyanophenyl)ethanol: 16.94 minutes, (S)-1-(4'-cyanophenyl)ethanol: 19.63 minutes.

[Example 55] Production of (R)-1-(4'-cyanophenyl)ethanol by asymmetric transfer hydrogenation of 4'-cyanoacetophenone using a catalyst (catalyst 16) containing a (R,R)-Ts-DENEB complex adsorbed on activated carbon (A2, granular)

The target (R)-1-(4'-cyanophenyl)ethanol was produced by the same operation as in Example 39 except that 4'-cyanoacetophenone (0.29 g, 2.0 mmol) was used as a raw material, and the heating and stirring time was changed to 5 hours. Table 25 shows the reaction conversion rate and the optical purity calculated from the GC analysis results.

[Comparative Example 14] Production of (R)-1-(4'-cyanophenyl)ethanol by asymmetric transfer hydrogenation of 4'-cyanoacetophenone using a (R,R)-Ts-DENEB complex

The target (R)-1-(4'-cyanophenyl)ethanol was produced by the same operation as in Comparative Example 2 except that 4'-cyanoacetophenone (0.29 g, 2.0 mmol) was used as a raw material, and the heating and stirring time was changed to 5 hours. Table 25 shows the reaction conversion rate and the optical purity calculated from the GC analysis results.

[Comparative Example 15] Production of (R)-1-(4'-cyanophenyl)ethanol by asymmetric transfer hydrogenation of 4'-cyanoacetophenone using a (R,R)-Ts-DENEB complex in the presence of formic acid-triethylamine

The target (R)-1-(4'-cyanophenyl)ethanol was produced by the same operation as in Comparative Example 3 except that 4'-cyanoacetophenone (0.29 g, 2.0 mmol) was used as a raw material, and the heating and stirring time was changed to 1 hour. Table 25 shows the reaction conversion rate and the optical purity calculated from the GC analysis results.

Table 25: Reaction conversion rate and the optical purity calculated from the GC analysis results

| | Catalyst | Solvent | Reductant | Reaction Time (Hour) | Conversion Rate (%) | Optical Purity (%ee) |
|---|---|---|---|---|---|---|
| Example 54 | Ts-DENEB/A ctivated Carbon C2 (Granular) | Water-Ethanol | Potassium Formate | 5 | 93.4 | 77.5 |
| Example 55 | Ts-DENEB/A ctivated Carbon A2 (Granular) | Water-Ethanol | Potassium Formate | 5 | 54.3 | 65.1 |
| Comparative Example 14 | Ts-DENEB | Water-Ethanol | Potassium Formate | 5 | 99.2 | 79.1 |
| Comparative Example 15 | Ts-DENEB | N/A | Formic Acid-Triethylamine | 1 | 99.8 | 86.4 |

The effects on the reaction conversion rate and optical purity in the production of (R)-1-(4'-tert-butylphenyl)ethanol by the asymmetric transfer hydrogenation of 4'-tert butyl acetophenone caused by the catalyst of the present invention were examined. Table 26 shows the results.

[Example 56] Production of (R)-1-(4'-tert butylphenyl)ethanol by asymmetric transfer hydrogenation of 4'-tert butyl acetophenone using a catalyst (catalyst 17) containing a (R,R)-Ts-DENEB complex adsorbed on activated carbon (C2, granular)

The target (R)-1-(4'-tert-butylphenyl)ethanol was produced by the same operation as in Example 40 except that 4'-tert butyl acetophenone (0.35 g, 2.0 mmol) was used as a raw material, distilled water (10 mL) and ethanol (10 mL) were used as solvents, and the heating and stirring time was changed to 5 hours. Table 26 shows the reaction conversion rate and the optical purity calculated from the GC analysis results.

GC retention time (measurement condition 4); 4'-tert butyl acetophenone: 20.38 minutes, (R)-1-(4'-tert butylphenyl)ethanol: 27.94 minutes, (S)-1-(4'-tert butylphenyl)ethanol: 29.81 minutes.

[Example 57] Production of (R)-1-(4'-tert butylphenyl)ethanol by asymmetric transfer hydrogenation of 4'-tert butyl acetophenone using a catalyst (catalyst 16) containing a (R,R)-Ts-DENEB complex adsorbed on activated carbon (A2, granular)

The target (R)-1-(4'-tert-butylphenyl)ethanol was produced by the same operation as in Example 39 except that 4'-tert butyl acetophenone (0.35 g, 2.0 mmol) was used as a raw material, distilled water (10 mL) and ethanol (10 mL) were used as solvents, and the heating and stirring time was changed to 5 hours. Table 26 shows the reaction conversion rate and the optical purity calculated from the GC analysis results.

[Comparative Example 16] Production of (R)-1-(4'-tert butylphenyl)ethanol by asymmetric transfer hydrogenation of 4'-tert butyl acetophenone using a (R,R)-Ts-DENEB complex

The target (R)-1-(4'-tert-butylphenyl)ethanol was produced by the same operation as in Comparative Example 2 except that 4'-tert-butyl acetophenone (0.35 g, 2.0 mmol) was used as a raw material, distilled water (10 mL) and ethanol (10 mL) were used as solvents, and the heating and stirring time was changed to 5 hours. Table 26 shows the reaction conversion rate and the optical purity calculated from the GC analysis results.

[Comparative Example 17] Production of (R)-1-(4'-tert butylphenyl)ethanol by asymmetric transfer hydrogenation of 4'-tert butyl acetophenone using a (R,R)-Ts-DENEB complex in the presence of formic acid-triethylamine

The target (R)-1-(4'-tert-butylphenyl)ethanol was produced by the same operation as in Comparative Example 3 except that 4'-tert butyl acetophenone (0.35 g, 2.0 mmol) was used as a raw material, distilled water (10 mL) and ethanol (10 mL) were used as solvents, and the heating and stirring time was changed to 3 hours. Table 26 shows the reaction conversion rate and the optical purity calculated from the GC analysis results.

Table 26: Reaction conversion rate and the optical purity calculated from the GC analysis results

| | Catalyst | Solvent | Reductant | Reaction Time (Hour) | Conversion Rate (%) | Optical Purity (%ee) |
|---|---|---|---|---|---|---|
| Example 56 | Ts-DENEB/Act ivated Carbon C2 (Granular) | Water-Ethanol | Potassium Formate | 5 | 99.7 | 97.3 |
| Example 57 | Ts-DENEB/Act ivated Carbon A2 (Granular) | Water-Ethanol | Potassium Formate | 5 | 77.4 | 95.2 |
| Comparative Example 16 | Ts-DENEB | Water-Ethanol | Potassium Formate | 5 | 78.6 | 96.5 |
| Comparative Example 17 | Ts-DENEB | N/A | Formic Acid-Triethylamin e | 3 | 98.5 | 97.7 |

The effects on the reaction conversion rate and optical purity in the production of (S)-pinacolyl alcohol by the asymmetric transfer hydrogenation of pinacolin caused by the catalyst of the present invention were examined. Table 27 shows the results.

[Example 58] Production of (S)-pinacolyl alcohol by asymmetric transfer hydrogenation of pinacolin using a catalyst (catalyst 17) containing a (R,R)-Ts-DENEB complex adsorbed on activated carbon (C2, granular)

The target (S)-pinacolyl alcohol was produced by the same operation as in Example 40 except that the catalyst was 181.8 mg (0.02 mmol, 1.0 mol%), pinacolin (0.20 g, 2.0 mmol) was used as a raw material, and the heating and stirring time was changed to 7 hours. Table 27 shows the reaction conversion rate and the optical purity calculated from the GC analysis results.

GC retention time (measurement condition 5); pinacolin: 3.12 minutes, (S)-pinacolyl alcohol: 9.13 minutes, (R)-pinacolyl alcohol: 9.69 minutes.

[Example 59] Production of (S)-pinacolyl alcohol by asymmetric transfer hydrogenation of pinacolin using a catalyst (catalyst 16) containing a (R,R)-Ts-DENEB complex adsorbed on activated carbon (A2, granular)

The target (S)-pinacolyl alcohol was produced by the same operation as in Example 39 except that the catalyst was 194.0 mg (0.02 mmol, 1.0 mol%), pinacolin (0.20 g, 2.0 mmol) was used as a raw material, and the heating and stirring time was changed to 7 hours. Table 27 shows the reaction conversion rate and the optical purity calculated from the GC analysis results.

[Comparative Example 18] Production of (S)-pinacolyl alcohol by asymmetric transfer hydrogenation of pinacolin using a (R,R)-Ts-DENEB complex

The target (S)-pinacolyl alcohol was produced by the same operation as in Comparative Example 2 except that the catalyst was 13.0 mg (0.02 mmol, 1.0 mol%), pinacolin (0.20 g, 2.0 mmol) was used as a raw material, and the heating and stirring time was changed to 7 hours. Table 27 shows the reaction conversion rate and the optical purity calculated from the GC analysis results.

[Comparative Example 19] Production of (S)-pinacolyl alcohol by asymmetric transfer hydrogenation of pinacolin using a (R,R)-Ts-DENEB complex in the presence of formic acid-triethylamine

The target (S)-pinacolyl alcohol was produced by the same operation as in Comparative Example 3 except that the catalyst was 13.0 mg (0.02 mmol, 1.0 mol%), pinacolin (0.20 g, 2.0 mmol) was used as a raw material, and the heating and stirring time was changed to 7 hours. Table 27 shows the reaction conversion rate and the optical purity calculated from the GC analysis results.

Table 27: Reaction conversion rate and the optical purity calculated from the GC analysis results

| | Catalyst | Solvent | Reductant | Reaction Time (Hour) | Conversion Rate (%) | Optical Purity (%ee) |
|---|---|---|---|---|---|---|
| Example 58 | Ts-DENEB/A ctivated Carbon C2 (Granular) | Water-Ethanol | Potassium Formate | 7 | 89.5 | 71.0 |
| Example 59 | Ts-DENEB/A ctivated Carbon A2 (Granular) | Water-Ethanol | Potassium Formate | 7 | 93.8 | 75.1 |
| Comparative Example 18 | Ts-DENEB | Water-Ethanol | Potassium Formate | 7 | 34.7 | 45.6 |
| Comparative Example 19 | Ts-DENEB | N/A | Formic Acid-Triethylamine | 7 | 57.0 | 47.3 |

As described above, it has been found that in the production of specific alcohols, the use of a complex supported on activated carbon gives an optical purity higher than that of conventional homogeneous complexes.

<Analysis 4> $^1$H NMR analysis in the coexistence of a (R,R)-Ts-DENEB complex and a $\pi$-conjugated compound

To an NMR sample tube, (R,R)-Ts-DENEB (20.0 mg, 0.0308 mmol) and a predetermined amount of pyrene were added, and then 0.7 mL of deuterated chloroform containing 0.05% of trimethylsilane was added and completely dissolved. When $^1$H NMR of the obtained solutions was compared as shown in Fig. 3, for the solutions containing pyrene, it was observed that the peak (4.5 ppm to 6.2 ppm) attributed to the protons around the aromatic ring on ruthenium shifted to the high magnetic field side. From this result, it has been inferred that when (R,R)-Ts-DENEB and the $\pi$-conjugated compound coexist, an interaction occurs between the aromatic ring on ruthenium and the $\pi$-conjugated compound.

## Claims

**1.** A catalyst comprising activated carbon adsorbed with a ruthenium complex, represented by the following general formula (1-1) and/or (1-2),

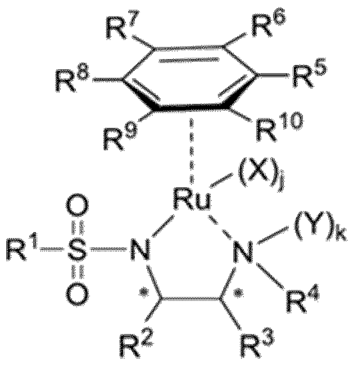

j = k = 1

(1-1)

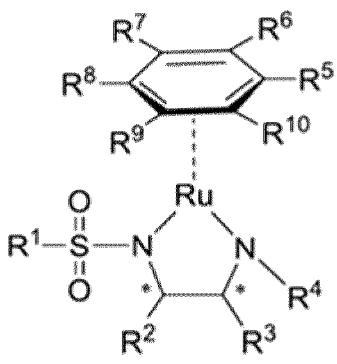

(1-2)

wherein

a solid line indicates a single bond, a double line indicates a double bond, and a broken line indicates a coordination bond;
Ru represents a ruthenium atom, N represents a nitrogen atom, S represents a sulfur atom, and O represents an oxygen atom;
* represents an asymmetric carbon atom;
j and k are integers of 0 or 1, where j+k does not become 1, and X represents an anionic group and Y represents

a hydrogen atom;
$R^1$ represents a linear or branched alkyl group having 1 to 10 carbon atoms; a 10-camphalyl group; an aryl group which may be substituted with an alkyl group having 1 to 10 carbon atoms, an alkyl halide group having 1 to 10 carbon atoms, a halogen atom, a cyano group (-CN), an amino group, an alkylamino group ($-NR^{11}R^{12}$), a 5-membered or 6-membered cyclic amino group, an acylamino group ($-NH-CO-R^{11}$), a hydroxyl group, an alkoxy group ($-OR^{11}$), an acyl group ($-CO-R^{11}$), a carboxyl group, an alkoxy carbonyl group ($-COOR^{11}$), a phenoxycarbonyl group, or an alkylthio group ($-SR^{11}$); or an aralkyl group which may be substituted with an alkyl group having 1 to 10 carbon atoms;
$R^{11}$ and $R^{12}$ each independently represent a hydrogen atom; an alkyl group having 1 to 10 carbon atoms; or a cycloalkyl group having 3 to 10 carbon atoms;
$R^2$ and $R^3$ each independently represent a hydrogen atom; or a phenyl group which may be substituted with an alkyl group having 1 to 10 carbon atoms, an alkoxy group having 1 to 10 carbon atoms, or a halogen atom, or $R^2$ and $R^3$ are bonded to each other to form a 4- to 8-membered cycloalkane ring with the carbon atom to which $R^2$ and $R^3$ are bonded;
$R^4$ represents a hydrogen atom; or a linear or branched alkyl group having 1 to 10 carbon atoms which may have a substituent;
$R^5$ to $R^{10}$ each independently represent a hydrogen atom; a linear or branched alkyl group having 1 to 10 carbon atoms; a hydroxyl group; or a linear or branched alkoxy group having 1 to 10 carbon atoms; and
$R^4$ and $R^5$ may be bonded to each other to form a cross-linking site of a divalent group represented by the following formula (W) to form a cross-linking site (W),

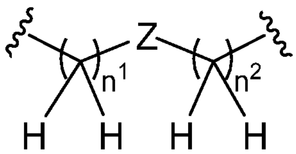

(W)

where

a wavy line section at a carbon chain terminal containing $n^1$ is bonded to a carbon atom of an arene moiety instead of $R^5$ in the formulas (1-1) and (1-2), and a wavy line section at a carbon chain terminal containing $n^2$ in the formula (W) is bonded to a nitrogen atom of an amine moiety instead of $R^4$ in the formulas (1-1) and (1-2);
Z represents a methylene group or an oxygen atom;
$n^1$ is an integer of 1 or 2; and
$n^2$ is an integer of 1, 2, or 3.

**2.** The catalyst according to claim 1, wherein

$R^4$ and $R^5$ are bonded to each other to form the cross-linking site of the divalent group represented by the above formula (W),
Z is an oxygen atom,
$n^1$ is 1, and
$n^2$ is 2.

**3.** The catalyst according to claim 2, wherein

$R^1$ is a 4-methylphenyl group or a methyl group,
$R^2$ and $R^3$ are phenyl groups,
$R^6$, $R^7$, $R^9$, and $R^{10}$ are hydrogen atoms, and
$R^8$ is a methyl group.

**4.** The catalyst according to claim 1, wherein

$R^4$ and $R^5$ do not form the cross-linking site of the divalent group represented by the above formula (W), and $R^4$ is a hydrogen atom.

**5.** The catalyst according to claim 4, wherein

$R^1$ is a 4-methylphenyl group, a 2,3,4,5,6-pentafluorophenyl group, a methyl group, an isobutyl group, a benzyl group, a 2',5'-dimethylbenzyl group, or a 10-camphalyl group,
$R^2$ and $R^3$ are phenyl groups or bonded to each other to form a cyclohexane ring, and
$R^5$, $R^6$, $R^7$, $R^8$, $R^9$ and $R^{10}$ are hydrogen atoms, methyl groups, or isopropyl groups.

**6.** The catalyst according to any one of claims 1 to 5, wherein a total mass of the ruthenium complex is 0.1% by mass or more and 25% by mass or less based on a total mass of the activated carbon.

**7.** The catalyst according to any one of claims 1 to 6, wherein the activated carbon has a specific surface area of 800 $m^2/g$ or more and 2000 $m^2/g$ or less.

**8.** A method for producing a reduction product, comprising: a step of reducing an organic compound in the presence of the catalyst according to any one of claims 1 to 7 and a hydrogen donor.

**9.** A method for producing an optically active alcohol, comprising: a step of reducing a carbonyl group of a carbonyl compound in the presence of the catalyst according to any one of claims 1 to 7 and a hydrogen donor.

**10.** A method for producing an optically active amine, comprising: a step of reducing an imino group of an imine compound in the presence of the catalyst according to any one of claims 1 to 7 and a hydrogen donor.

**11.** The production method according to any one of claims 8 to 10, wherein the hydrogen donor is at least one selected from the group consisting of formic acid, an alkali metal formate, an alcohol having a hydrogen atom at an $\alpha$-position carbon atom of a hydroxyl group substituted carbon, and hydrogen gas.

FIG.1

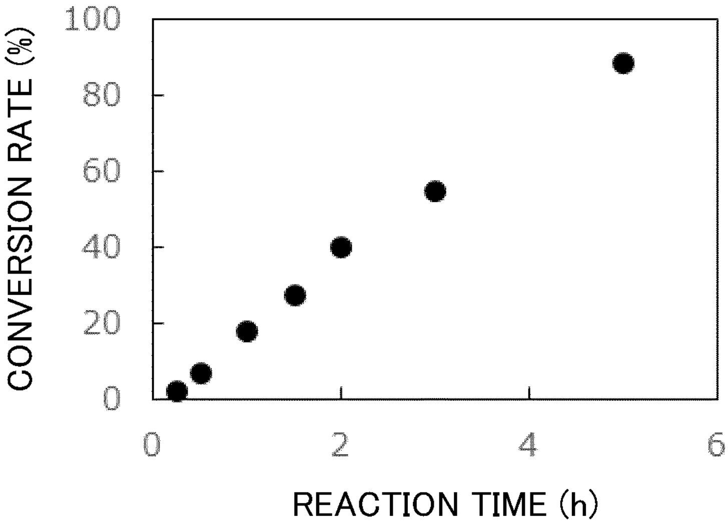
CONVERSION RATE (%)
100
80
60
40
20
0
0
2
4
6
REACTION TIME (h)

FIG.2

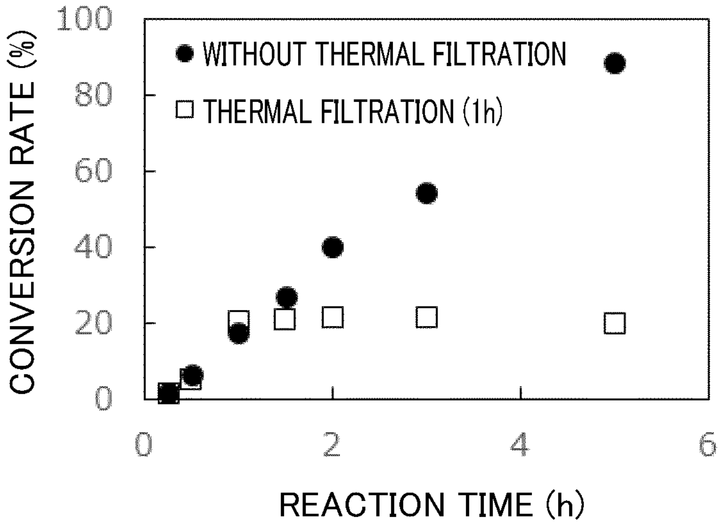
CONVERSION RATE (%)
100
80
60
40
20
0
● WITHOUT THERMAL FILTRATION
□ THERMAL FILTRATION (1h)
0
2
4
6
REACTION TIME (h)

# FIG.3

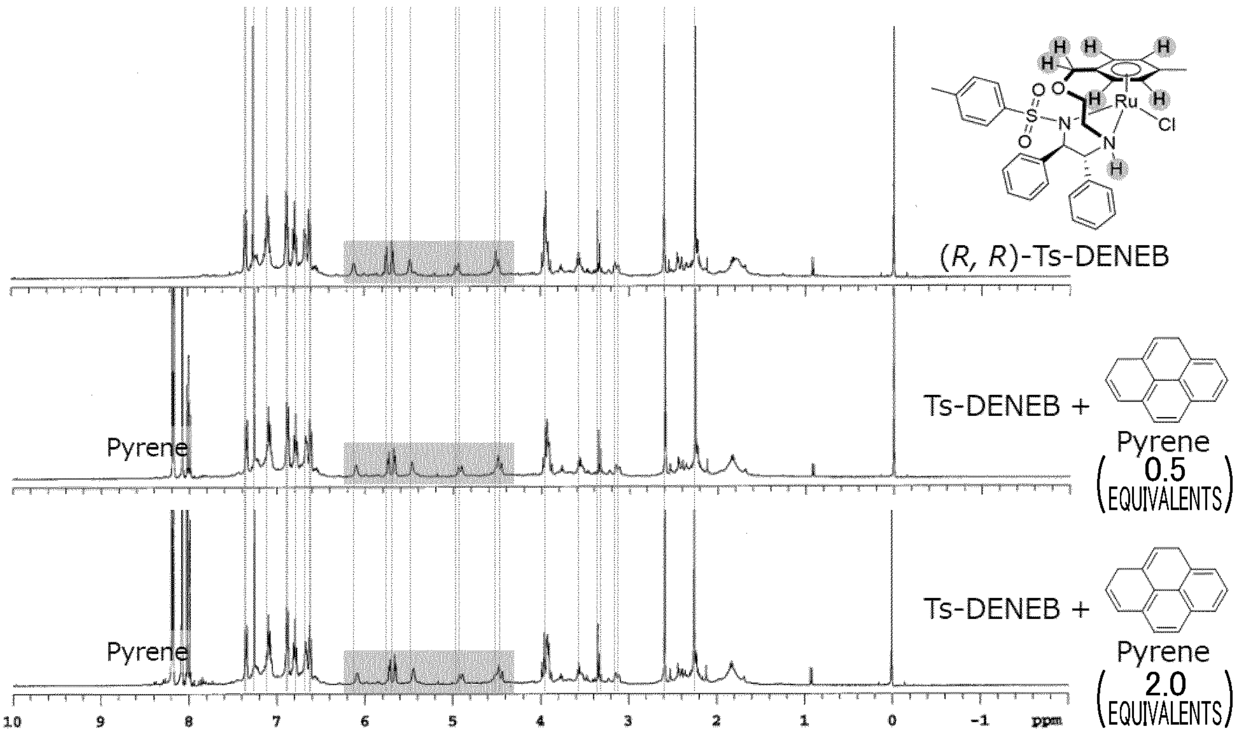
(R, R)-Ts-DENEB
Ts-DENEB +
Pyrene
(0.5 EQUIVALENTS)
Ts-DENEB +
Pyrene
(2.0 EQUIVALENTS)
Pyrene
Pyrene
10
9
8
7
6
5
4
3
2
1
0
-1
ppm

FIG.4

CATALYST 17

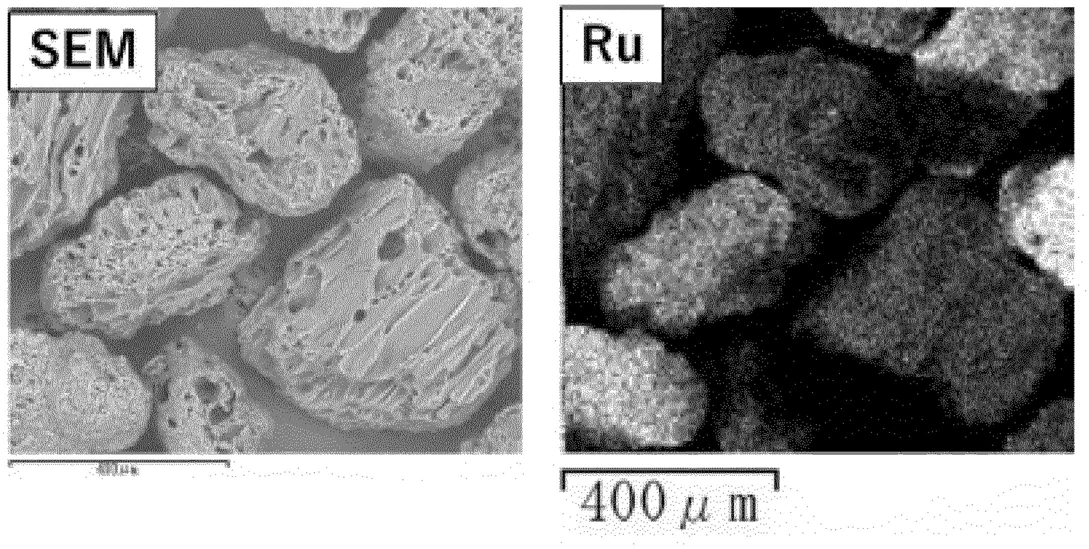


CATALYST 20

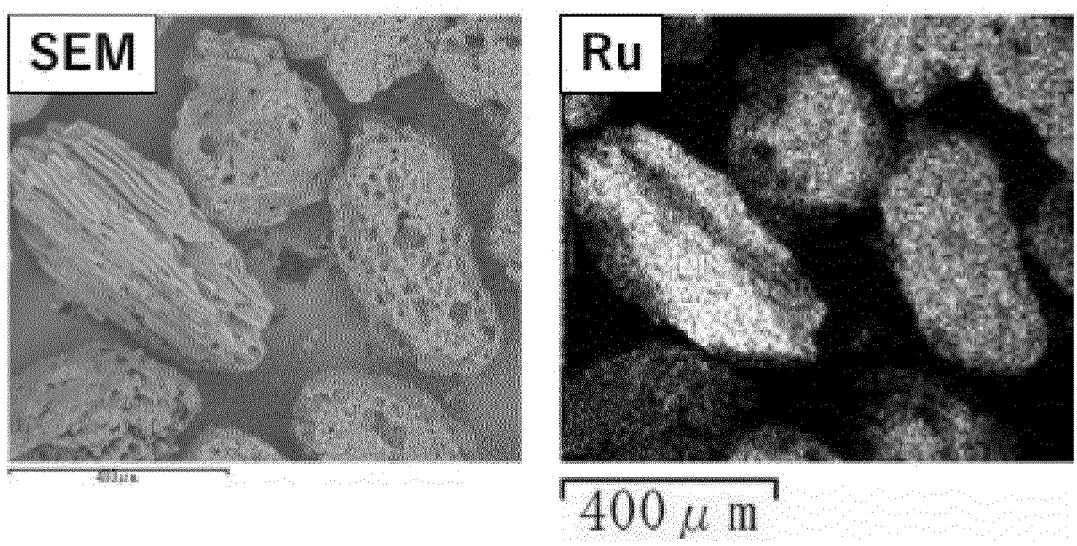

**INTERNATIONAL SEARCH REPORT**

International application No. PCT/JP2021/007899

A. CLASSIFICATION OF SUBJECT MATTER
B01J 31/22(2006.01)i; C07B 53/00(2006.01)i; C07C 29/159(2006.01)i; C07C 31/125(2006.01)i; C07C 33/22(2006.01)i; C07C 33/46(2006.01)i; C07C 41/26(2006.01)i; C07C 43/23(2006.01)i; C07C 253/30(2006.01)i; C07C 255/53(2006.01)i; C07B 61/00(2006.01)n
FI: B01J31/22 Z; C07C33/22; C07C29/159; C07C33/46; C07C43/23 B; C07C41/26; C07C255/53; C07C253/30; C07C31/125; C07B53/00 A; C07B61/00 300
According to International Patent Classification (IPC) or to both national classification and IPC

B. FIELDS SEARCHED
Minimum documentation searched (classification system followed by classification symbols)
B01J31/22; C07B53/00; C07C29/159; C07C31/125; C07C33/22; C07C33/46; C07C41/26; C07C43/23; C07C253/30; C07C255/53; C07B61/00

Documentation searched other than minimum documentation to the extent that such documents are included in the fields searched

| | |
|---|---|
| Published examined utility model applications of Japan | 1922-1996 |
| Published unexamined utility model applications of Japan | 1971-2021 |
| Registered utility model specifications of Japan | 1996-2021 |
| Published registered utility model applications of Japan | 1994-2021 |

Electronic data base consulted during the international search (name of data base and, where practicable, search terms used)
CAplus/REGISTRY (STN)

C. DOCUMENTS CONSIDERED TO BE RELEVANT

| Category* | Citation of document, with indication, where appropriate, of the relevant passages | Relevant to claim No. |
|---|---|---|
| Y | WO 2012/147944 A1 (TAKASAGO INTERNATIONAL CORPORATION) 01 November 2012 (2012-11-01) examples, claims | 1-3, 6-9, 11 |
| Y | CN 102050688 A (INSTITUTE OF CHEMISTRY, CHINESE ACADEMY OF SCIENCES) 11 May 2011 (2011-05-11) claims, paragraph [0063], examples | 1, 4-8, 10, 11 |
| Y | JP 2000-256247 A (DICEL CHEMICAL INDUSTRIES, LTD.) 19 September 2000 (2000-09-19) claims, paragraphs [0008], [0009], examples | 1-11 |

☒ Further documents are listed in the continuation of Box C. ☒ See patent family annex.

* Special categories of cited documents:
"A" document defining the general state of the art which is not considered to be of particular relevance
"E" earlier application or patent but published on or after the international filing date
"L" document which may throw doubts on priority claim(s) or which is cited to establish the publication date of another citation or other special reason (as specified)
"O" document referring to an oral disclosure, use, exhibition or other means
"P" document published prior to the international filing date but later than the priority date claimed
"T" later document published after the international filing date or priority date and not in conflict with the application but cited to understand the principle or theory underlying the invention
"X" document of particular relevance; the claimed invention cannot be considered novel or cannot be considered to involve an inventive step when the document is taken alone
"Y" document of particular relevance; the claimed invention cannot be considered to involve an inventive step when the document is combined with one or more other such documents, such combination being obvious to a person skilled in the art
"&" document member of the same patent family

| Date of the actual completion of the international search | Date of mailing of the international search report |
|---|---|
| 20 April 2021 (20.04.2021) | 11 May 2021 (11.05.2021) |
| Name and mailing address of the ISA/<br>Japan Patent Office<br>3-4-3, Kasumigaseki, Chiyoda-ku,<br>Tokyo 100-8915, Japan | Authorized officer<br><br>Telephone No. |

Form PCT/ISA/210 (second sheet) (January 2015)

**INTERNATIONAL SEARCH REPORT**

International application No.
PCT/JP2021/007899

C (Continuation). DOCUMENTS CONSIDERED TO BE RELEVANT

| Category* | Citation of document, with indication, where appropriate, of the relevant passages | Relevant to claim No. |
|---|---|---|
| Y | WO 90/15791 A1 (JGC CORPORATION) 27 December 1990 (1990-12-27) examples, claims | 1-11 |
| A | WO 2016/056669 A1 (TAKASAGO INTERNATIONAL CORPORATION) 14 April 2016 (2016-04-14) entire text | 1-11 |

Form PCT/ISA/210 (continuation of second sheet) (January 2015)

**INTERNATIONAL SEARCH REPORT**
Information on patent family members

International application No.
PCT/JP2021/007899

| Patent Documents referred in the Report | Publication Date | Patent Family | Publication Date |
|---|---|---|---|
| WO 2012/147944 A1 | 01 Nov. 2012 | US 9073827 B2<br>examples, claims<br>EP 2703386 A1<br>CN 103502208 A | |
| CN 102050688 A | 11 May 2011 | (Family: none) | |
| JP 2000-256247 A | 19 Sep. 2000 | US 6506943 B1<br>column 2, lines 25-41, examples, claims<br>EP 1078907 A1<br>CN 1296468 A<br>KR 10-2001-0043429 A | |
| WO 90/15791 A1 | 27 Dec. 1990 | US 5324700 A<br>examples, claims<br>EP 429675 A1 | |
| WO 2016/056669 A1 | 14 Apr. 2016 | US 2018/0264449 A1<br>EP 3205656 A1 | |

Form PCT/ISA/210 (patent family annex) (January 2015)

## REFERENCES CITED IN THE DESCRIPTION

*This list of references cited by the applicant is for the reader's convenience only. It does not form part of the European patent document. Even though great care has been taken in compiling the references, errors or omissions cannot be excluded and the EPO disclaims all liability in this regard.*

### Patent documents cited in the description


- JP 3040353 B
- JP 5718178 B
- JP 5727127 B
- WO 2016056669 A


### Non-patent literature cited in the description


- *Acc. Chem. Res.,* 1997, vol. 30, 97-102
- *J. Am. Chem. Soc.,* 2011, vol. 133, 14960-14963
- *Org. Lett.,* 2004, vol. 6, 169-172
- *Tetrahedron Asymmetry,* 1998, vol. 9, 2015-2018
- *Chem. Commun.,* 2010, vol. 46, 8145-8147
- *Org. Lett.,* 1999, vol. 1, 713-715
- *Org. Lett.,* 2002, vol. 4, 2369-2371
- *Org. Process Res. Dev.,* 2018, vol. 22, 1580-1585